# EUROPÄISCHE PATENTSCHRIFT

(11) **EP 3 273 997 B1**
(45) Veröffentlichungstag und Bekanntmachung des Hinweises auf die Patenterteilung: **17.07.2019**
(21) Anmeldenummer: 16723344.4
(22) Anmeldetag: 12.05.2016
(51) Int. Cl.: A61K 31/717, A61K 31/164, A61K 9/00, A61K 9/12, A61P 27/14

(54) **ZUSAMMENSETZUNG FÜR DIE BEHANDLUNG VON RHINITIS**
COMPOSITIONS FOR THE TREATMENT OF COLD
COMPOSITIONS POUR LE TRAITEMENT DES RHINITES

(30) Priorität: 06.07.2015 DE 102015008522; 20.08.2015 DE 102015113802
(43) Veröffentlichungstag der Anmeldung: 31.01.2018
(73) Patentinhaber: Maria Clementine Martin Klosterfrau Vertriebsgesellschaft mbH, 50670 Köln (DE)
(72) Erfinder: UNKAUF, Markus, 80538 München (DE); VESTWEBER, Anna-Maria, 51399 Burscheid (DE); GALÀN SOÙSA, José, 13465 Berlin (DE)
(74) Vertreter: Strehlke, Ingo Kurt
(86) Internationale Anmeldenummer: PCT/EP2016/060725
(87) Internationale Veröffentlichungsnummer: WO 2017/005398

(56) Entgegenhaltungen:
- WO-A1-2010/015253
- WO-A1-2016/026600
- WO-A2-2010/066437
- DE-A1- 10 326 899
- DE-U1-202006 005 924
- DE-U1-202006 011 920
- STOZKOWSKA WIESLAWA ET AL: "Investigation of some topical formulations containing dexpanthenol", ACTA POLONIAE PHARMACEUTICA - DRUG RESE, POLISH PHARMACEUTICAL SOCIETY, WARZSAW, PL, vol. 61, no. 6, 1 November 2004 (2004-11-01), pages 433-437, XP009190958, ISSN: 0001-6837

## Beschreibung

Die vorliegende Erfindung betrifft das medizinisch-therapeutische Gebiet der Behandlung von Rhinitiden.

Insbesondere betrifft die vorliegende Erfindung eine Zusammensetzung, welche sich vorzugsweise für die topische, insbesondere nasale, bevorzugt intranasale Applikation eignet, insbesondere für die Behandlung von Rhinitiden, und die Verwendung dieser erfindungsgemäßen Zusammensetzung sowie eine die erfindungsgemäße Zusammensetzung enthaltende Applikationsvorrichtung.

Unter einer Rhinitis (synonym gelegentlich auch als Nasenkatarrh, Schnupfen oder Koryza bezeichnet) versteht man im Rahmen der vorliegenden Erfindung insbesondere eine akute oder chronische Entzündung der Nasenschleimhäute, wobei die Rhinitis insbesondere infektiösen (z. B. viralen oder bakteriellen), allergischen oder pseudoallergischen Ursprungs sein kann. Am Häufigsten tritt eine Rhinitis im Rahmen einer sogenannten Erkältung auf.

Neben einer Unterscheidung von akuter Rhinitis einerseits und chronischer Rhinitis andererseits unterscheidet man auch verschiedene Formen von Rhinitiden, so beispielsweise die folgenden Rhinitisformen: *Rhinitis acuta*, *Rhinitis atrophicans*, *Rhinitis allergica*, *Rhinitis hypertrophica*, *Rhinitis medicamentosa*, *Rhinitis pseudomembranacea*, *Rhinitis sicca*, *Rhinitis vasomotorica* und die umweltbedingte Rhinitis.

Bei der sogenannten akuten Rhinitis (*Rhinitis acuta*), d. h. dem gewöhnlichen Schnupfen, handelt es sich in der Regel um einen im Allgemeinen harmlosen Infekt der Nasenschleimhäute und damit um eine infektiöse Rhinitis, welche meist viral, insbesondere durch eine Vielzahl von Viren (insbesondere Rhinoviren und/oder Adenoviren), gelegentlich aber auch bakteriell ausgelöst werden kann. Hauptmerkmal einer akuten Rhinitis ist eine sogenannte laufende Nase und eine Verstopfung der Nase durch die Anschwellung der Schleimhäute.

Insgesamt sind mehr als 200 "Schnupfenviren" als mögliche Auslöser einer viralen Rhinitis bekannt, wie sie üblicherweise im Rahmen einer gewöhnlichen Erkältung auftreten kann. Im Rahmen einer Erkältung, welche im Allgemeinen mit einer Rhinitis beginnt, verschwindet aber die *Rhinitis acuta* im Allgemeinen. Gelegentlich kann es jedoch mitunter auch zu einer Chronifizierung kommen, welche oftmals mit einer Volumenzunahme der Schleimhäute unter anderem im Bereich der Nasenmuscheln mit Behinderung der Nasenatmung einhergeht.

Eine Rhinitis (d. h. eine Entzündung der Nasenschleimhaut), insbesondere eine akute Rhinitis, kann oftmals gleichzeitig auch mit einer Entzündung der Schleimhaut der Nasennebenhöhlen (Sinusitis) vorliegen; in einem solchen Fall spricht man auch von einer Rhinosinusitis. Auch kann eine Rhinitis, insbesondere eine akute Rhinitis, gleichzeitig auch mit einer Entzündung des Rachens (Pharyngitis) vorliegen; in einem solchen Fall spricht man auch von einer Rhinopharyngitis.

Für weitergehende Einzelheiten zum Begriff der Rhinitis kann insbesondere verwiesen werden auf Pschyrembel, Medizinisches Wörterbuch, 257. Auflage, Seiten 1331/1332, insbesondere Stichwörter "*Rhinitis*", "*Rhinitis allergica*", "*Rhinitis atrophicans*", "*Rhinitis hyperplastica*", "*Rhinitis pseudomembranacea*", "*Rhinitis sicca*" und "*Rhinitis vasomotorica*".

Da es also eine Vielzahl verschiedener Typen von Viren gibt, welche eine akute virale Rhinitis auslösen können, und da eine Vielzahl von Ursachen für das Auftreten einer Rhinitis existieren, können Rhinitiden, insbesondere akute Rhinitiden, im Allgemeinen nicht kausal, sondern nur symptomatisch, insbesondere topisch, behandelt werden.

Zu diesem Zweck kommen in den meisten Fällen intranasal zu applizierende Zusammensetzungen zur Anwendung, welche sogenannte Sympathomimetika (synonym auch als "Abschweller", "Dekongestiva" oder dergleichen bezeichnet), vorzugweise alpha-Sympathomimetika (wie z. B. Xylometazolin und Oxymetazolin oder deren physiologisch verträgliche bzw. unbedenkliche Salze), enthalten.

Diese Sympathomimetika führen zwar aufgrund ihrer vasokonstriktorischen Eigenschaften nach lokaler bzw. topischer Anwendung in der Nase zunächst zu einer Nasenschleimhautabschwellung, bewirken jedoch bei wiederholten Anwendung oftmals eine Austrocknung der Nasenschleimhäute, einhergehend mit entzündlichen Irritationen der Nasenschleimhäute (was nicht selten zu einer erhöhten Infektionsgefahr führt, da die Nasenschleimhaut im ausgetrockneten und entzündeten Zustand nicht mehr ihre Schutz- und Filterfunktion im vollen Umfang aufrechterhalten kann und folglich Krankheitserreger ungehindert in die Atemwege gelangen können).

Darüber hinaus haben die Sympathomimetika zum Teil auch schwerwiegende systemische (z. B. kardiovaskuläre) Nebenwirkungen, insbesondere im Fall von Überdosierung oder bei der Therapierung bestimmter Patienten (z. B. Kleinkindern, Patienten mit kardiovaskulären Vorerkrankungen etc.).

Weiterhin besteht bei dem Einsatz von Sympathomimetika das Problem, dass diese bei häufiger oder längerfristiger Anwendung zu einer gewissen Abhängigkeit führen, da die Nasenschleimhaut nur noch unter Verwendung von Sympathomimetika und nicht mehr von alleine abschwillt. Dies äußert sich dann als dauerhaft "verstopfte" Nase, was auch als *Rhinitis medicamentosa* bezeichnet wird.

Um den zahlreichen Nebenwirkungen der Sympathomimetika zumindest teilweise entgegenzuwirken, werden in für die intranasale Applikation bestimmte sympathomimetikahaltige Zusammensetzungen bisweilen weitere Wirk- bzw. Inhaltsstoffe inkorporiert, beispielsweise pflanzliche Extrakte oder pflanzliche Inhaltsstoffe, befeuchtende oder schleimhautpflegende Zusätze, Antihistaminika, Salze etc. (vgl. beispielsweise DE 195 41 919 A1, DE 195 49 421 A1 und DE 103 56 248 A1).

Darüber hinaus sind aus dem Stand der Technik für die Behandlung von Rhinitiden auch sympathomimetikafreie, für die intranasale Applikation bestimmte Zusammensetzungen bekannt, z. B. Zusammensetzungen auf Basis ätherischer Öle, welche aber oftmals keine ausreichende Wirksamkeit in Bezug auf eine effiziente Behandlung von Rhinitiden aufweisen. Darüber hinaus weisen derartige Zusammensetzungen ebenfalls nicht immer eine optimale Verträglichkeit auf. Insbesondere pflanzliche Stoffe, wie beispielsweise ätherische Öle, können zu Schleimhautreizungen bzw. Unverträglichkeitsreaktionen führen.

Zudem zeichnen sich zahlreiche, aus dem Stand der Technik bekannte Zusammensetzungen, welche im Rahmen der Behandlung von Rhinitiden verabreicht werden, lediglich durch kurze Wirkdauern aus.

So betrifft die WO 2010/015253 A1 eine pharmazeutische Zubereitung zur prophylaktischen bzw. kurativen Behandlung von trockener Nasenschleimhaut sowie von Rhinitiden, wobei die Zubereitung neben Ectoin bzw. mindestens einem Ectoinderivat zudem Pantothenol bzw. mindestens ein Pantothenolderivat aufweist.

Weiterhin betrifft die DE 20 2006 011 920 U1 eine pharmazeutische Zusammensetzung für die intranasale Applikation, welche bevorzugt in Form eines Nasensprays oder in Form von Nasentropfen vorliegen soll, wobei die Zusammensetzung einerseits Natriumchlorid und andererseits mindestens eine Schleimdroge, insbesondere in Form von deren Extrakt, insbesondere auf Basis von Isländischem Moos (*Lichen islandicus*), enthalten soll.

Darüber hinaus betrifft die DE 20 2006 005 924 U1 eine pharmazeutische Zusammensetzung, welche insbesondere in Form einer flüssigen Formulierung für die intranasale Applikation vorliegt, wobei die Zusammensetzung Natriumchlorid einerseits und Myrrhe andererseits aufweisen soll.

Die WO 2010/066437 A2 betrifft eine wässrige pharmazeutische Zusammensetzung, welche Dexpanthenol und Natriumchlorid bei einer Osmolalität der Zusammensetzung von 200 bis 400 mosmol/kg aufweisen soll.

Weiterhin betrifft die DE 103 26 899 A1 eine kosmetische oder dermatologische Zubereitung, welche Kaliumsorbat und Stabilisierungsmittel enthält. Die Stabilisierungsmittel werden ausgewählt aus der Gruppe von mikrokristalliner Cellulose bzw. Talkum oder, für den Fall, dass die Zubereitung auf einer Wasser-in-Öl- oder einer Wasser-in-Silikonöl-Emulsion basiert, Lipiden mit einer Grenzflächenspannung von größer oder gleich 10 mN/m.

Zudem betrifft die wissenschaftliche Publikation gemäß Stozkowska W. et al.: "Investigations of some topical formulations containing dexpanthenol", erschienen in Acta Poloniae Pharmaceutica - Drug Research, 2004, Vol. 61, No. 6, p. 433-437, Zusammensetzungen zur Behandlung von Schleimhäuten, welche Dexpanthenol sowie Hydroxyethylcellulose, Phosphatpuffer, Sorbitol und Konservierungsmittel enthalten. Als Koservierungsmittel werden dabei Parabene eingesetzt. Die Zusammensetzungen können insbesondere als Lutschtabletten oder als Hydrogele ausgebildet sein.

Das der vorliegenden Erfindung zugrundeliegende Problem besteht daher in der Bereitstellung einer für die topische, insbesondere nasale, bevorzugt intranasale Applikation, insbesondere für die Behandlung von Rhinitiden (darüber hinaus aber auch von Sinusitiden, Rhinosinusitiden oder Rhinopharyngitiden), geeigneten Zusammensetzung, insbesondere pharmazeutischen Zusammensetzung, welche die zuvor geschilderten Nachteile des Standes der Technik zumindest weitgehend vermeidet oder aber wenigstens abschwächt.

Insbesondere soll eine solche Zusammensetzung eine gegenüber herkömmlichen, für die Behandlung von Rhinitiden mittels topischer bzw. intranasaler Applikation bestimmten, pharmazeutischen Präparaten eine verbesserte Wirkeffizienz und/oder ein verbessertes Nebenwirkungsprofil aufweisen. Darüber hinaus soll insbesondere auch die Wirkdauer verlängert sein.

Zur Lösung der zuvor geschilderten Aufgabe schlägt die vorliegende Erfindung - gemäß dem ersten Aspekt der vorliegenden Erfindung - eine Zusammensetzung nach Anspruch 1 vor; weitere, insbesondere vorteilhafte Ausgestaltungen der erfindungsgemäßen Zusammensetzung sind Gegenstand der diesbezüglichen Unteransprüche.

Weiterhin betrifft die vorliegende Erfindung - gemäß einem zweiten Aspekt der vorliegenden Erfindung - eine die erfindungsgemäße Zusammensetzung enthaltende Applikationsvorrichtung gemäß dem diesbezüglichen Vorrichtungsanspruch; weitere, insbesondere vorteilhafte Ausgestaltungen der erfindungsgemäßen Applikationsvorrichtung sind Gegenstand der diesbezüglichen Unteransprüche.

Es versteht sich im Zusammenhang mit den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt aufgeführt sind, selbstverständlich auch in Bezug auf die übrigen Erfindungsaspekte entsprechend gelten, ohne dass dies einer gesonderten Erwähnung bedarf.

Bei allen nachstehend genannten relativen bzw. prozentualen gewichtsbezogenen Angaben, insbesondere Mengenangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltsstoffe, insbesondere wie nachfolgend definiert, stets zu 100% bzw. 100 Gew.-% ergänzen bzw. addieren; dies versteht sich aber für den Fachmann von selbst.

Weiterhin gilt, dass der Fachmann - anwendungsbezogen oder einzelfallbedingt - von den nachfolgend angeführten Gewichts-, Mengen- und Bereichsangaben abweichen kann, ohne dass er den Rahmen der vorliegenden Erfindung verlässt.

Zudem gilt, dass alle im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr nachfolgend im Detail erläutert.

Gegenstand der vorliegenden Erfindung - gemäß einem **ersten** Aspekt der vorliegenden Erfindung - ist somit eine Zusammensetzung zur Verwendung bei der topischen nasalen, bevorzugt intranasalen Applikation für die Behandlung von Rhinitiden,
wobei die Zusammensetzung in Kombination und jeweils in wirksamen, insbesondere pharmazeutisch wirksamen Mengen enthält:
(a) Pantothenol, vorzugsweise Dexpanthenol (D-Pantothenol), oder dessen physiologisch unbedenkliche Ester und/oder Pantothensäure oder deren physiologisch unbedenkliche Salze (Komponente (a)), wobei die Zusammensetzung die Komponente (a) in einer Menge im Bereich von 0,001 bis 20 Gew.-%, bezogen auf die Zusammensetzung, enthält; und
(b) Cellulose und/oder ein physiologisch unbedenkliches Cellulosederivat aus der Gruppe von Carboxymethylcellulose, Methylcellulose, Ethylcellulose, Hydroxyethylcellulose, Hydroxyethylmethylcellulose und/oder Hydroxypropylmethylcellulose (Komponente (b)), wobei die Zusammensetzung die Komponente (b) in einer Menge im Bereich von 0,0001 bis 5 Gew.-%, bezogen auf die Zusammensetzung, enthält;
(c) mindestens ein Konservierungsmittel (Komponente (c)), wobei die Zusammensetzung die Komponente (c) in einer Menge im Bereich von 0,001 bis 10 Gew.-%, bezogen auf die Zusammensetzung, enthält und wobei die Komponente (c) ausgewählt ist aus der Gruppe von Sorbinsäure und/oder deren pharmazeutisch verträglichen Salzen (Sorbaten);
wobei die Zusammensetzung gegenüber den Zellen der Nasenschleimhaut hyperton ausgebildet ist.

Überraschend wurde im Rahmen der vorliegenden Erfindung gefunden, dass auf Basis der erfindungsgemäßen Kombination von Inhaltsstoffen, welche Panthenol bzw. Pantothensäure einerseits sowie Cellulose bzw. physiologisch unbedenkliche/verträgliche Cellulosederivate andererseits umfasst, Zusammensetzungen zur nasalen Applikation im Rahmen der Behandlung von Rhinitiden bereitgestellt werden können, welche gegenüber dem Stand der Technik ein verbessertes Wirkungsprofil und darüber hinaus verringerte Nebenwirkungen aufweisen.

Die vorliegende Erfindung ist mit zahlreichen Vorteilen und Besonderheiten verbunden, welche nachfolgend in nicht beschränkender Weise diskutiert sind und als Indiz für die Patentfähigkeit zu werten sind.

Auf Basis der erfindungsgemäßen Zusammensetzung wird ein hochwirksames Mittel zur Behandlung von Rhinitiden bereitgestellt, welches ohne Anwesenheit von Sympathomimetika eine hervorragende Wirksamkeit auch im Hinblick auf ein Abschwellen der Nasenschleimhäute besitzt, so dass die zuvor geschilderten, unerwünschten Nebenwirkungen von Sympathomimetika vermieden werden können. Insbesondere kann einem Austrocknen der Nasenschleimhaut und einer Abhängigkeit von Sympathomimetika (*Rhinitis medicamentosa*), insbesondere auch bei längerfristiger Anwendung der Zusammensetzung, vorgebeugt werden. Darüber hinaus birgt die erfindungsgemäße Zusammensetzung zumindest im Wesentlichen keine Gefahr von systemischen Nebenwirkungen, wie es bei der Anwesenheit von Sympathomimetika der Fall ist. Durch die Bereitstellung der erfindungsgemäßen Zusammensetzung in Form einer gegenüber der Nasenschleimhaut hypertonen Lösung können die adstringierenden Eigenschaften der erfindungsgemäßen Zusammensetzung noch weiterführend verstärkt werden.

Weiterhin wirkt die erfindungsgemäße Kombination von Inhaltsstoffen entzündungshemmend, so dass Entzündungen der Nasenschleimhaut, wie sie üblicherweise mit Rhinitiden einhergehen, effizient gelindert werden. Die erfindungsgemäße Kombination von Inhaltsstoffen - nämlich Panthenol bzw. Pantothensäure einerseits und Cellulose bzw. Cellulosederivaten andererseits, vorzugsweise in hypertoner Lösung - wirkt zudem nicht nur entzündungshemmend bzw. antiinflammatorisch, sondern besitzt darüber hinaus auch schleimhautprotektive bzw. wundheilungsfördernde Eigenschaften. Auch wird die Nasenschleimhaut gepflegt und befeuchtet, so dass einer Austrocknung und Borkenbildung entgegengewirkt wird. Denn - ohne sich hierbei auf diese Theorie beschränken zu wollen - bilden Cellulose bzw. Cellulosederivate eine Art schützenden Film auf der Nasenschleimhaut aus, was wiederum einem Austrocknen der Nasenschleimhaut vorbeugt und so die entzündungshemmende Wirkung von Pantothenol bzw. Pantothensäure noch weiterführend verstärkt. Pantothenol bzw. Pantothensäure und Cellulose bzw. Cellulosederivate ergänzen sich somit in synergistischer Weise in der erfindungsgemäßen Zusammensetzung.

Darüber hinaus hat sich im Rahmen der vorliegenden Erfindung überraschend gezeigt, dass durch den Einsatz von Cellulose bzw. Cellulosederivaten, insbesondere Hydroxypropylmethylcellulose (HPMC), - ohne sich hierbei auf diese Theorie beschränken zu wollen - die Freisetzung bzw. der Wirkeintritt der übrigen Inhaltsstoffe retardiert bzw. verzögert wird, so dass diese über einen längeren Zeitraum ihre Wirkung auf der Nasenschleimhaut entfalten können. Auf Basis des Einsatzes von Cellulose bzw. Cellulosederivaten wird somit insgesamt die Wirkdauer der erfindungsgemäßen Zusammensetzungen verbessert bzw. verlängert.

Weiterhin ist die erfindungsgemäße Zusammensetzung in Bezug auf ihre Anwendungseigenschaften bzw. die Handhabung im Rahmen der insbesondere intranasalen Applikation verbessert, da sie unmittelbar nach der Anwendung an der Nasenschleimhaut haften bleibt und so einem Herausrinnen bzw. Heraustropfen der Zusammensetzung aus der Nase vorgebeugt wird.

Insgesamt wird somit auf Basis des synergistischen Zusammenwirkens der Inhaltsstoffe der erfindungsgemäßen Zusammensetzung, insbesondere von Pantothenol bzw. Pantothensäure einerseits und Cellulose bzw. Cellulosederivaten andererseits, eine hervorragende antirhinitische Wirkung erzielt, welche darüber hinaus auch äußerst lang anhält. Durch Applikation der erfindungsgemäßen Zusammensetzung kann Symptomen von Rhinitiden, d.h. dem sogenannten Fließschnupfen, einer verstopften Nase, gereizter und entzündeter Nasenschleimhaut, effizient entgegengewirkt werden. Dabei ist die erfindungsgemäße Zusammensetzung dennoch zumindest im Wesentlichen frei von Nebenwirkungen und darüber hinaus komfortabel in der Handhabung.

Insbesondere ist die erfindungsgemäße Zusammensetzung ohne bzw. frei von Sympathomimetika und/oder Dekongestiva ausgebildet bzw. enthält diese Substanzen nicht.

Durch die optionale Inkorporierung weiterer Wirk- bzw. Inhaltsstoffe lassen sich Wirkprofil und Stabilitätsverhalten der erfindungsgemäßen Zusammensetzung kontrollieren bzw. gezielt einstellen.

Wie zuvor angeführt, enthält die erfindungsgemäße Zusammensetzung als Komponente (a) Pantothenol, vorzugsweise Dexpanthenol (D-Pantothenol), bzw. dessen physiologisch unbedenkliche Ester und/oder Pantothensäure bzw. deren physiologisch unbedenkliche Salze.

Der Wirkstoff Pantothenol gehört in chemischer Hinsicht zu den Polyolen und Amiden und ist in physiologischer Hinsicht ein Provitamin (d. h. ein Präkursor eines Vitamins der Vitamin B-Gruppe), welches im Körper zu Pantothensäure (Vitamin B5) umgewandelt wird. Der Wirkstoff Pantothensäure wiederum ist ein Bestandteil des Coenzyms A und spielt damit eine wesentliche Rolle im (Schleim-)-Hautstoffwechsel.

Wie zuvor bereits ausgeführt, entfaltet die Komponente (a) (d. h. Pantothenol, vorzugsweise Dexpanthenol, oder dessen physiologisch unbedenkliche Ester bzw. Pantothensäure oder deren physiologisch unbedenkliche Salze) in der erfindungsgemäßen Zusammensetzung bei deren bestimmungsgemäßer Verwendung in Kombination mit den übrigen Inhaltsstoffen eine schleimhautprotektive und wundheilungsfördernde Wirkung und wirkt darüber hinaus auch eigenständig entzündungshemmend bzw. antiinflammatorisch. In synergistischer Kombination mit Cellulose bzw. Cellulosederivaten wird folglich Entzündungen der Nasenschleimhaut, wie sie bei Rhinitiden stets auftreten, effizient entgegengewirkt. Zudem wirkt die Komponente (a) in Bezug auf die Nasenschleimhaut pflegend und befeuchtend, so dass auf diese Weise eine gute Befeuchtung der Nasenschleimhaut bewirkt wird bzw. einer Austrocknung der Nasenschleimhaut und einer Borkenbildung entgegengewirkt wird, wobei die vorgenannten Effekte durch Komponente (b) noch verstärkt werden.

Auch wird durch die Komponente (a) in Zusammenwirkung mit der Komponente (b) eine verbesserte Nasenatmung erreicht. In synergistischer Zusammenwirkung mit der Komponente (b) wird folglich insgesamt eine antirhinitische Wirkung erzielt und zudem den Symptomen von Rhinitiden effizient entgegengewirkt, insbesondere auch dem Fließschnupfen (Rhinorrhö) (d. h. die erfindungsgemäße Zusammensetzung bzw. Kombination besitzt - wie zuvor angeführt - auch ein antirhinorrhöisches Wirkprofil bzw. Wirkpotential).

Erfindungsgemäß ist es bevorzugt, wenn die Zusammensetzung als Komponente (a) Pantothenol, vorzugsweise Dexpanthenol (D-Pantothenol), oder dessen physiologisch unbedenkliche Ester, besonders bevorzugt Dexpanthenol (D-Pantothenol), enthält.

Auch die eingesetzte Menge an Komponente (a) bzw. Pantothenol bzw. Pantothensäure kann in weiten Bereichen variieren.

Besonders gute Ergebnisse im Hinblick auf die Wirksamkeit der erfindungsgemäßen Zusammensetzung werden erzielt, wenn die Zusammensetzung die Komponente (a) in einer Menge im Bereich von 0,005 bis 15 Gew.-%, vorzugsweise 0,01 bis 10 Gew.-%, bevorzugt 0,1 bis 1 Gew.-%, besonders bevorzugt 0,2 bis 0,8 Gew.-%, bezogen auf die Zusammensetzung, enthält.

Darüber hinaus enthält die erfindungsgemäße Zusammensetzung - wie zuvor angeführt - Cellulose bzw. mindestens ein physiologisch unbedenkliches bzw. verträgliches Cellulosederivat:
Bei Cellulose handelt es sich um ein unverzweigtes Polysaccharid auf Basis von beta-D-Glucose-Einheiten bzw. Cellobioseeinheiten (zwei durch beta-1,4-glykosidische Bindungen verknüpfte Glucose-Moleküle), welche durch beta-1,4-glykosidische Bindungen miteinander verknüpft sind. Die Länge der Polysaccharide bzw. die Anzahl der die Cellulose bildenden Monomere kann in weiten Bereichen variieren, insbesondere besteht Cellulose aus mehreren hundert bis zehntausend beta-D-Glucose-Einheiten. Cellulose ist Hauptbestandteil von pflanzlichen Zellwänden und darüber hinaus die am häufigsten vorkommende organische Verbindung.

Unter Cellulosederivaten hingegen werden im Rahmen der vorliegenden Erfindung insbesondere durch polymeranaloge Reaktionen chemisch modifizierte Cellulosen verstanden.

Insbesondere kann es sich bei den erfindungsgemäß eingesetzten Cellulosederivaten um Celluloseether, welche vollständig oder partiell an den Wasserstoffatomen der Hydroxy-Gruppen der Cellulose mit Aryl- bzw. (Ar)alkyl-Gruppen substituiert sind, handeln. Die Aryl- bzw. (Ar)alkyl-Gruppen können darüber hinaus funktionelle nicht-, an- oder kationische Gruppe enthalten.

Darüber hinaus können erfindungsgemäß auch Celluloseester, welche vollständig oder partiell an den Wasserstoffatomen der Hydroxy-Gruppen der Cellulose mit organischen oder anorganischen Säureresten substituiert sind, eingesetzt werden. Celluloseester können insbesondere durch eine Veresterung von Cellulose bzw. cellulosehaltigen Materialien mit organischen bzw. anorganischen Säuren oder Säurederivaten hergestellt werden.

In Bezug auf weiterführende Ausführungen zu Celluloseestern bzw. Celluloseethern kann darüber hinaus verwiesen werden auf RÖMPP Chemie Lexikon, 10. Auflage, Thieme-Verlag, Stuttgart/New York, Seite 639 bis 640, Stichworte: "Celluloseester" und "Celluloseether", sowie auf die dort referierte Literatur.

Im Hinblick auf den Einsatz von Cellulosederivaten ist es erfindungsgemäß vorgesehen, dass die Komponente (b) ausgewählt ist aus Carboxymethylcellulose, Methylcellulose, Ethylcellulose, Hydroxyethylcellulose, Hydroxyethylmethylcellulose und/oder Hydroxypropylmethylcellulose. Besonders bevorzugt ist als Komponente (b) Hydroxypropylmethylcellulose (HPMC) eingesetzt.

Unter Hydroxypropylmethylcellulose (synonym auch als HPMC, Hypromellose oder Methocel bezeichnet) werden im Rahmen der vorliegenden Erfindung Celluloseether verstanden, welche auf partiell bzw. vollständig alkylsubstituierten Cellulosen basieren. Hydroxypropylmethylcellulose kann in verschiedenen Polymerisationsgraden und verschiedenen Substitutionsgraden vorliegen. Zur Herstellung von Hydroxypropylmethylcellulose wird Cellulose in heißer Natronlauge gequollen und bei 50 bis 80 °C mit Propylenoxid unter hohem Methylchlorid-Druck umgesetzt. Im Stand der Technik wird Hydroxypropylmethylcellulose vor allem als Verdickungs-, Binde-, Klebe-, Dispergier-, Suspendier-, Emulgier-, Flockungs-, Quell-, Gleit- oder Wasserrückhaltemittel sowie als Schutzkolloid in verschiedenen technischen Bereichen eingesetzt.

Für weitergehende Einzelheiten zu dem Inhaltsstoff Hydroxypropylmethylcellulose kann beispielsweise verwiesen werden auf RÖMPP Chemie Lexikon, Thieme-Verlag, Stuttgart/New York, 10. Auflage, Band 4, 1996, Seite 2644 bis 2645, Stichwort: "Methylcellulose", sowie auf die dort referierte Literatur.

Im Rahmen der vorliegenden Erfindung hat sich überraschend gezeigt, dass der Einsatz von Hydroxypropylmethylcellulose in der erfindungsgemäßen Zusammensetzung für die topische, insbesondere nasale, bevorzugt intranasale Applikation zur Behandlung von Rhinitiden insgesamt zu einer verbesserten Wirksamkeit, insbesondere einer verlängerten Wirkdauer, auch der übrigen Inhaltsstoffe der Zusammensetzung, insbesondere der Komponente (a), führt. Ohne sich hierbei auf diese Theorie beschränken zu wollen, werden durch den Einsatz von Hydroxypropylmethylcellulose die übrigen Wirk- bzw. Inhaltsstoffe retardiert bzw. zeitverzögert freigesetzt, so dass die Wirkstoffe über einen längeren Zeitraum ihre Wirkung am Zielort entfalten können. Darüber hinaus wird durch die Cellulose bzw. die physiologisch verträglichen Cellulosederivate, insbesondere die Hydroxypropylmethylcellulose, eine Art Schutzfilm auf der Nasenschleimhaut ausgebildet, welcher eine Befeuchtung der Nasenschleimhaut sicherstellt und so weiterführend Entzündungen vorbeugt und die Heilung der Nasenschleimhaut insgesamt beschleunigt. Insbesondere die Wirkung des Pantothenols bzw. der Pantothensäure wird durch den Einsatz von Hydroxypropylmethylcellulose verstärkt.

Was darüber hinaus die eingesetzte Menge an Cellulose bzw. physiologisch verträglichen Cellulosederivaten anbelangt, so kann diese in weiten Bereichen variieren:
Im Rahmen der vorliegenden Erfindung hat es sich als besonders vorteilhaft erwiesen, wenn die erfindungsgemäße Zusammensetzung die Komponente (b) in einer Menge im Bereich von 0,0001 bis 2 Gew.-%, vorzugsweise 0,001 bis 1,5 Gew.-%, bevorzugt 0,005 bis 1 Gew.-%, besonders bevorzugt 0,01 bis 0,1 Gew.-%, noch mehr bevorzugt 0,02 bis 0,05 Gew.-%, bezogen auf die Zusammensetzung, enthält.

Neben den eingesetzten Mengen der vorgenannten Komponenten als solchen ist zudem auch deren gewichtsbezogenes Mengenverhältnis zueinander für die Wirksamkeit der erfindungsgemäßen Zusammensetzung von Bedeutung. Gemäß einer bevorzugten Ausführungsform der vorliegenden Erfindung kann es vorgesehen sein, dass die Zusammensetzung die Komponente (a) und die Komponente (b) in einem gewichtsbezogen Verhältnis von (a) : (b) im Bereich von 1 : 1 bis 500 : 1, insbesondere 1,25 : 1 bis 100 : 1, vorzugsweise 1,5 : 1 bis 75 : 1, bevorzugt 2 : 1 bis 50 : 1, besonders bevorzugt 10 : 1 bis 30 : 1, enthält.

Dennoch kann es einzelfallbedingt oder anwendungsbezogen erforderlich sein, von den vorgenannten Werten abzuweichen, ohne dass der Rahmen der vorliegenden Erfindung verlassen ist, was im Ermessen des Fachmanns liegt.

Gemäß einer besonders bevorzugten Ausführungsform der vorliegenden Erfindung kann es zudem vorgesehen sein, dass die Zusammensetzung als Komponente (c) mindestens ein Konservierungsmittel enthält.

Der Einsatz eines Konservierungsmittels ist in mehrfacher Hinsicht vorteilhaft. Einerseits hat sich gezeigt, dass die Stabilität der Zusammensetzung, insbesondere die Stabilität der Inhaltsstoffe, noch weiterführend gesteigert werden kann. Weiterhin wird die Zusammensetzung auf dieser Basis vor mikrobiellem Befall geschützt. Darüber hinaus hat sich überraschend gezeigt, dass auch die Wirksamkeit der übrigen Inhaltsstoffe bzw. der Inhaltsstoffe mit pharmazeutisch relevanter Wirkung durch den Einsatz eines Konservierungsmittels weiterführend gesteigert werden kann.

Was die Auswahl des Konservierungsmittels bzw. der Komponente (c) anbelangt, ist es erfindungsgemäß vorgesehen, dass dieses bzw. diese ausgewählt ist aus der Gruppe von Sorbinsäure und/oder deren pharmazeutisch verträglichen Salzen (Sorbaten).

Besonders gute Ergebnisse werden im Rahmen der vorliegenden Erfindung erzielt, wenn das Konservierungsmittel ein Alkali- oder Erdalkalisorbat, vorzugsweise ein Alkalisorbat, bevorzugt Kaliumsorbat, ist. Durch den Einsatz von Alkali- bzw. Erdalkalisorbat, insbesondere Kaliumsorbat, lassen sich die übrigen Inhaltsstoffe besonders gut stabilisieren. Einerseits bleiben die erfindungsgemäßen Zusammensetzungen bei Einsatz von Alkali- bzw. Erdalkalisorbat, insbesondere Kaliumsorbat, als Konservierungsmittel besonders lange frei von mikrobiologischem Befall und andererseits ist die Lagerstabilität insgesamt erhöht, d. h. die Zusammensetzungen bleiben auch über einen langen Zeitraum frei von Eintrübungen bzw. Ausflockungen der Inhaltsstoffe und sind somit länger verwendbar. Insbesondere wird speziell durch den Einsatz von Alkali- bzw. Erdalkalisorbat, insbesondere Kaliumsorbat, einem vorzeitigen Abbau der übrigen Inhaltsstoffe der erfindungsgemäßen Zusammensetzung entgegengewirkt.

Was die eingesetzte Menge des Konservierungsmittels anbelangt, so kann diese in weiten Bereichen variieren. Besonders gute Ergebnisse werden erzielt, wenn die Zusammensetzung die Komponente (c) in einer Menge im Bereich von 0,005 bis 5 Gew.-%, vorzugsweise im Bereich 0,01 bis 1 Gew.-%, bevorzugt im Bereich von 0,1 bis 0,5 Gew.-%, bezogen auf die Zusammensetzung, enthält.

Neben den eingesetzten Mengen der Komponente (c) bzw. Konservierungsmittels als solchen ist darüber hinaus auch das Mengenverhältnis des Konservierungsmittels zu den übrigen Komponenten, d. h. insbesondere dem Pantothenol bzw. der Pantothensäure und der Cellulose bzw. dem Cellulosederivat, in Bezug auf die Wirksamkeit der erfindungsgemäßen Zusammensetzung von Bedeutung.

Erfindungsgemäß hat es sich als besonders vorteilhaft erwiesen, wenn die Zusammensetzung die Komponente (a) und die Komponente (c) in einem gewichtsbezogen Verhältnis von (a) : (c) im Bereich von 250: 1 bis 1 : 10, insbesondere 100 : 1 bis 1 : 1, vorzugsweise 50 : 1 bis 1 : 1, bevorzugt 25 : 1 bis 1,25 : 1, besonders bevorzugt 10 : 1 bis 2 : 1, ganz besonders bevorzugt 5 : 1 bis 2 : 1, enthält.

Darüber hinaus ist es erfindungsgemäß bevorzugt, wenn die Zusammensetzung die Komponente (b) und die Komponente (c) in einem gewichtsbezogen Verhältnis von (b) : (c) im Bereich von 1 : 500 bis 10: 1, insbesondere 1 : 250 bis 5 : 1, vorzugsweise 1 : 200 bis 2 : 1, bevorzugt 1 : 50 bis 1 : 1, besonders bevorzugt 1 : 20 bis 1 : 2, noch mehr bevorzugt 1 : 15 bis 1 : 5, enthält.

Zudem hat es sich im Rahmen der vorliegenden Erfindung als vorteilhaft erwiesen, wenn die erfindungsgemäße Zusammensetzung (d) mindestens ein chemisches Puffersystem, insbesondere in Form von Puffersalz(en), enthält.

Das chemische Puffersystem, insbesondere in Form von Puffersalz(en), dient im Rahmen der vorliegenden Erfindung insbesondere zur Stabilisierung der erfindungsgemäßen Zusammensetzung. Insbesondere führt die Einstellung eines konstanten pH-Werts mittels des Puffersystems überraschenderweise dazu, dass einem unerwünschten Abbau der Wirkstoffe, insbesondere der Komponenten (a) und (b), bei Lagerung auch über einen längeren Zeitraum effizient entgegengewirkt wird. Auf diese Weise wird die Lagerfähigkeit der erfindungsgemäßen Zusammensetzung verbessert.

Zum Begriff des chemischen Puffers bzw. des chemischen Puffersystems kann insbesondere verwiesen werden auf RÖMPP Lexikon Chemie, 10. Auflage, Georg-Thieme-Verlag, Stuttgart/New York, Band 5, 1998, Seiten 3618/3619, Stichwort: "*Puffer*", sowie auf die dort referierte Literatur.

Was die eingesetzte Menge an chemischem Puffer anbelangt, so kann diese in weiten Bereichen variieren. Es hat sich jedoch als vorteilhaft erwiesen, wenn die Zusammensetzung das chemische Puffersystem, bezogen auf die Zusammensetzung und berechnet als Summe aller Bestandteile des chemischen Puffersystems, in einer Menge im Bereich von 0,001 bis 4 Gew.-%, insbesondere 0,01 bis 3 Gew.-%, vorzugsweise 0,05 bis 2 Gew.-%, bevorzugt 0,1 bis 1,5 Gew.-%, besonders bevorzugt 0,2 bis 1 Gew.-%, enthält.

In diesem Zusammenhang ist es zudem erfindungsgemäß vorgesehen, dass das chemische Puffersystem zur Einstellung und/oder Konstanthaltung des pH-Werts der Zusammensetzung dient.

Was darüber hinaus die Auswahl eines geeigneten Puffersystems anbelangt, so hat es sich als besonders vorteilhaft erwiesen, wenn das chemische Puffersystem als ein Dihydrogenphosphat/Monohydrogenposphat-Puffersystem ("H₂PO₄⁻/ HPO₄²⁻-Puffer(system)" bzw. "Phosphatpuffer(system)"), insbesondere als ein Alkalidihydrogenphosphat/Alkalimonohydrogenposphat-Puffersystem, vorliegt, bevorzugt mit einem Dihydrogenphosphat/Monohydrogenposphat-Molverhältnis von größer 5 : 1, insbesondere im Bereich von 5 : 1 bis 200 : 1, vorzugsweise 6 : 1 bis 175 : 1, bevorzugt 7 : 1 bis 150 : 1, besonders bevorzugt 8 : 1 bis 125 : 1, ganz besonders bevorzugt 10 : 1 bis 100 : 1, noch mehr bevorzugt 20 : 1 bis 90 : 1.

Bei Verwendung dieses speziellen Puffersystems, insbesondere bei gleichzeitiger Einhaltung der vorgenannten Molverhältnisse, wird einem unerwünschten Abbau der Wirkstoffe bei Lagerung auch über einen längeren Zeitraum in besonderem Maße entgegengewirkt und wird die Lagerfähigkeit der erfindungsgemäßen Zusammensetzung in besonderem Maße verbessert.

Grundsätzlich kann der pH-Wert der erfindungsgemäßen Zusammensetzung in weiten Bereichen variieren. Erfindungsgemäß bevorzugt ist es, wenn die Zusammensetzung einen pH-Wert im Bereich von 4,5 bis 8,0, insbesondere im Bereich von 5,0 bis 6,5, vorzugsweise im Bereich von 5,0 bis 6,2, bevorzugt im Bereich von 5,0 bis 6,0, noch mehr bevorzugt im Bereich von 5,1 bis 6,0, ganz besonders bevorzugt im Bereich von 5,2 bis 5,9, am meisten bevorzugt im Bereich von 5,25 bis 5,85, aufweist. Gleichermaßen kann es vorgesehen sein, dass der pH-Wert der Zusammensetzung Wert im Bereich von 4,5 bis 8,0, insbesondere im Bereich von 5,0 bis 6,5, vorzugsweise im Bereich von 5,0 bis 6,2, bevorzugt im Bereich von 5,0 bis 6,0, noch mehr bevorzugt im Bereich von 5,1 bis 6,0, ganz besonders bevorzugt im Bereich von 5,2 bis 5,9, am meisten bevorzugt im Bereich von 5,25 bis 5,85, eingestellt und/oder konstant gehalten wird, vorzugsweise mittels mindestens eines chemischen Puffersystems.

Bei Einhaltung der vorgenannten pH-Wertebereiche wird einem unerwünschten Abbau der Wirkstoffe bei Lagerung auch über einen längeren Zeitraum in besonderem Maße entgegengewirkt und wird auch die Lagerfähigkeit der erfindungsgemäßen Zusammensetzung in besonderem Maße verbessert.

Im Rahmen der vorliegenden Erfindung kann die Bestimmung des pH-Werts mit dem Fachmann an sich bekannten Methoden erfolgen. Insbesondere kann die Bestimmung des pH-Werts gemäß der Methode nach Ph. Eur. [Pharmacopoea Europaea] 7. Ausgabe, Grundwerk 2011, Abschnitt 2.2.3, erfolgen.

Auch im Zusammenhang mit den eingesetzten Mengen des chemischen Puffersystems bzw. der Komponente (d) hat es sich als vorteilhaft erwiesen, wenn dieses bzw. diese in definierten Mengenverhältnissen zu den übrigen eingesetzten Komponenten (a) bis (c) eingesetzt wird.

Was das Verhältnis von Panthenol bzw. Pantothensäure zu chemischem Puffersystem anbelangt, so weist die erfindungsgemäße Zusammensetzung diese vorzugsweise in einem gewichtsbezogen Verhältnis von (a): (d) im Bereich von 1 : 100 bis 100 : 1, insbesondere 1 : 50 bis 50 : 1, vorzugsweise 1 : 20 bis 20 : 1, bevorzugt 1 : 10 bis 10 : 1, besonders bevorzugt 1 : 5 bis 2 : 1, ganz besonders bevorzugt 1 : 2 bis 1 : 1, auf.

Was darüber hinaus das gewichtsbezogene Verhältnis von Cellulose bzw. Cellulosederivat (b) zu dem chemischen Puffersystem (d) anbelangt, so liegt dieses gewichtsbezogene Verhältnis von (b) zu (d) vorzugsweise im Bereich von 1 : 1.000 bis 100 : 1, insbesondere 1 : 500 bis 10 : 1, vorzugsweise 1 : 200 bis 1 : 1, bevorzugt 1 : 150 bis 1 : 15, vorzugsweise 1 : 100 bis 1 : 30.

Schließlich kann es vorgesehen sein, dass die erfindungsgemäße Zusammensetzung das Konservierungsmittel gemäß Komponente (c) und das chemische Puffersystem gemäß Komponente (d) in einem gewichtsbezogen Verhältnis von (c) : (d) im Bereich von 1 : 500 bis 100 : 1, insbesondere 1 : 100 bis 5 : 1, vorzugsweise 1 : 20 bis 3 : 1, bevorzugt 1 : 10 bis 1 : 1, besonders bevorzugt 1 : 6 bis 1 : 2, enthält.

Darüber hinaus hat es sich im Rahmen der vorliegenden Erfindung als vorteilhaft erwiesen, wenn die Zusammensetzung als hyperosmolare Lösung vorliegt. Erfindungsgemäß ist es vorgesehen, dass die Zusammensetzung gegenüber den Zellen der Nasenschleimhaut hyperton ausgebildet ist.

In diesem Zusammenhang kann es insbesondere vorgesehen sein, dass die erfindungsgemäße Zusammensetzung als Komponente (e) mindestens einen physiologisch unbedenklichen bzw. verträglichen, osmotisch aktiven Stoff und/oder mindestens einen physiologisch unbedenklichen bzw. verträglichen Osmolyten enthält. Unter osmotisch aktiven Stoffen bzw. Osmolyten werden üblicherweise Substanzen verstanden, welche den osmotischen Zustand eines Systems, insbesondere von Zellen, beeinflussen können.

Durch die Bereitstellung der erfindungsgemäßen Zusammensetzung auf Basis einer hyperosmolaren Lösung kann die adstringierende Wirkung der erfindungsgemäßen Zusammensetzung bzw. können die abschwellenden Effekte noch signifikant verbessert werden. Denn durch den gegenüber den Zellen der Nasenschleimhaut erhöhten Gehalt an osmotisch aktiven Stoffen bzw. Osmolyten wird den Zellen der Nasenschleimhaut überschüssig gebundenes Wasser entzogen, so dass die Nasenschleimhaut abschwillt und die Nasenatmung verbessert wird. Darüber hinaus erfolgt eine Verbesserung der Bewegung der Flimmerhärchen, so dass Schleim- und Fremdkörper, wie beispielsweise Pollen oder Staub, besser abtransportiert werden können. Durch den erfindungsgemäßen Einsatz von Pantothenol bzw. Pantothensäure kann zudem verhindert werden, dass hypertone Lösungen im Rahmen der erfindungsgemäßen Verwendung zu einer Austrocknung der Nasenschleimhaut führen.

Was die speziell eingesetzten osmotisch aktiven Stoffe bzw. Osmolyte anbelangt, so sind diese variabel, sofern eine physiologische Verträglichkeit gewährleistet ist. Insbesondere kann es im Rahmen der vorliegenden Erfindung vorgesehen sein, dass die osmotisch aktiven Stoffe und/oder die Osmolyte ausgewählt sind aus der Gruppe von Ionen von physiologisch unbedenklichen bzw. verträglichen Salzen (d.h. Salz-Ionen), insbesondere Alkali-Ionen, insbesondere Natrium-Ionen und/oder Kalium-Ionen, und/oder Chlorid-Ionen, vorzugsweise Natrium-Ionen und Chlorid-Ionen; Zuckern, insbesondere Trehalose und/oder Saccharose; Polyolen, insbesondere Glycerin, und/oder Aminosäuren, insbesondere Glutaminsäure und/oder dessen Salzen, Prolin, (Hydroxy-) Ectoin und/oder Glycinbetain. Gemäß einer besonders bevorzugten Ausführungsform der vorliegenden Erfindung sind die osmotisch aktiven Stoffe bzw. die Osmolyte Natrium-Ionen und Chlorid-Ionen, insbesondere in Form von Natriumchlorid.

Auch die Menge der eingesetzten osmotisch aktiven Stoffe bzw. Osmolyte kann in weiten Bereichen variieren. Eine besonders gute Wirkung wird im Rahmen der vorliegenden Erfindung erzielt, wenn die Zusammensetzung physiologisch verträgliche, osmotisch aktive Stoffe und/oder physiologisch verträgliche Osmolyte in einer Menge im Bereich von 0,1 bis 25 Gew.-%, insbesondere 1 bis 15 Gew.-%, vorzugsweise 1 bis 15 Gew.-%, bevorzugt 1,5 bis 10 Gew.-%, besonders bevorzugt 2 bis 5 Gew.-%, bezogen auf die Zusammensetzung, enthält.

Gegenstand der vorliegenden Erfindung ist somit insbesondere eine Zusammensetzung, insbesondere pharmazeutische Zusammensetzung, welche für die topische, insbesondere nasale, bevorzugt intranasale Applikation, zur Behandlung von Rhinitiden geeignet ist und zuvor beschrieben wurde,
wobei die Zusammensetzung in Kombination und jeweils in wirksamen, insbesondere pharmazeutisch wirksamen Mengen enthält:
(a) Pantothenol, vorzugsweise Dexpanthenol (D-Pantothenol), oder dessen physiologisch unbedenkliche Ester und/oder Pantothensäure oder deren physiologisch unbedenkliche Salze (Komponente (a)), insbesondere in einer Menge im Bereich von 0,001 bis 20 Gew.-%, insbesondere 0,005 bis 15 Gew.-%, vorzugsweise 0,01 bis 10 Gew.-%, bevorzugt 0,1 bis 1 Gew.-%, besonders bevorzugt 0,2 bis 0,8 Gew.-%, bezogen auf die Zusammensetzung; und
(b) Cellulose und/oder physiologisch verträgliche Cellulosederivate (Komponente (b)), insbesondere in einer Menge im Bereich von 0,0001 bis 5 Gew.-%, insbesondere 0,0001 bis 2 Gew.-%, vorzugsweise 0,001 bis 1,5 Gew.-%, bevorzugt 0,005 bis 1 Gew.-%, besonders bevorzugt 0,01 bis 0,1 Gew.-%, noch mehr bevorzugt 0,02 bis 0,05 Gew.-%, bezogen auf die Zusammensetzung;
(c) gegebenenfalls mindestens ein Konservierungsmittel (Komponente (c)), insbesondere in einer Menge im Bereich von 0,001 bis 10 Gew.-%, insbesondere im Bereich von 0,005 bis 5 Gew.-%, vorzugsweise im Bereich 0,01 bis 1 Gew.-%, bevorzugt im Bereich von 0,1 bis 0,5 Gew.-%, bezogen auf die Zusammensetzung;
(d) gegebenenfalls mindestens ein chemisches Puffersystem (Komponente (d)), insbesondere in Form von Puffersalz(en), insbesondere in einer Menge im Bereich von 0,001 bis 4 Gew.-%, insbesondere 0,01 bis 3 Gew.-%, vorzugsweise 0,05 bis 2 Gew.-%, bevorzugt 0,1 bis 1,5 Gew.-%, besonders bevorzugt 0,2 bis 1 Gew.-%, bezogen auf die Zusammensetzung und berechnet als Summe aller Bestandteile des chemischen Puffersystems; und
(e) gegebenenfalls mindestens einen physiologisch unbedenklichen bzw. verträglichen, osmotisch aktiven Stoff und/oder physiologisch unbedenklichen bzw. verträglichen Osmolyten (Komponente (e)) in einer Menge im Bereich von 0,1 bis 25 Gew.-%, insbesondere 1 bis 20 Gew.-%, vorzugsweise 1 bis 15 Gew.-%, bevorzugt 1,5 bis 10 Gew.-%, besonders bevorzugt 2 bis 5 Gew.-%, bezogen auf die Zusammensetzung.

Darüber hinaus wird es im Rahmen der vorliegenden Erfindung bevorzugt, wenn die Zusammensetzung als wässrige Zusammensetzung vorliegt bzw. wässrig basiert bzw. wässrig formuliert ist. Insbesondere kann es vorgesehen sein, dass die Zusammensetzung in Form einer wässrigen Lösung oder wässrigen Solubilisierung vorliegt.

Was die eingesetzte Menge an Wasser anbelangt, so kann diese in weiten Bereichen variieren. Erfindungsgemäß ist es bevorzugt, wenn die Zusammensetzung Wasser, insbesondere gereinigtes Wasser, in einer Menge im Bereich von 60 bis 99 Gew.-%, insbesondere im Bereich von 70 bis 98 Gew.-%, bevorzugt im Bereich von 80 bis 97 Gew.-%, besonders bevorzugt im Bereich von 90 bis 96 Gew.-%, bezogen auf die Zusammensetzung, enthält.

Insbesondere kann es vorgesehen sein, dass die Zusammensetzung Wasser als pharmazeutisch verträglichen Träger (Exzipienten) enthält bzw. dass die Zusammensetzung wässrig basiert ausgebildet ist.

Im Zusammenhang mit der Wirksamkeit, insbesondere der abschwellenden Wirkung, hat es sich zudem als vorteilhaft erwiesen, wenn die Zusammensetzung eine Osmolalität im Bereich von 300 bis 600 mosm/kg, insbesondere im Bereich von 310 bis 550 mosm/kg, vorzugsweise im Bereich von 300 bis 525 mosm/kg, bevorzugt im Bereich von 325 bis 510 mosm/kg, besonders bevorzugt im Bereich von 350 bis 500 mosm/kg, aufweist.

Darüber hinaus ist es erfindungsgemäß bevorzugt, wenn die Zusammensetzung bei einer Temperatur von 20 °C und bei einem Druck von 1.013,25 mbar (Atmosphärendruck) eine relative Dichte, bezogen auf reines Wasser, im Bereich von 1,001 bis 1,5, insbesondere im Bereich von 1,001 bis 1,2, vorzugsweise im Bereich von 1,005 bis 1,15, bevorzugt im Bereich von 1,005 bis 1,1, aufweist.

Im Rahmen der vorliegenden Erfindung kann die Bestimmung der relativen Dichte der erfindungsgemäßen Zusammensetzung mit dem Fachmann an sich bekannten Methoden erfolgen. Insbesondere kann die Bestimmung der relativen Dichte der erfindungsgemäßen Zusammensetzung gemäß der Methode nach Ph.Eur. [Pharmacopoea Europaea] 7. Ausgabe, Grundwerk 2011, Abschnitt 2.2.5, erfolgen.

Im Zusammenhang mit der Handhabung bzw. Applikation der erfindungsgemäßen Zusammensetzung hat es sich zudem als vorteilhaft erwiesen, wenn diese bei einer Temperatur von 20 °C eine dynamische Viskosität von mindestens 1,1 mPas, insbesondere im Bereich von 1,1 bis 10⁵ mPas, vorzugsweise im Bereich von 1,2 bis 10⁴ mPas, besonders bevorzugt im Bereich von 1,3 bis 10³ mPas, noch mehr bevorzugt im Bereich von 1,5 bis 100 mPas, aufweist.

Im Rahmen der vorliegenden Erfindung kann die Bestimmung der dynamischen Viskosität mit dem Fachmann an sich bekannten Methoden erfolgen. Insbesondere kann die Methode gemäß Ph. Eur. [Pharmacopoea Europaea] 7. Ausgabe, Grundwerk 2011, Abschnitt 2.2.9 "Kapillarviskosimeter" [bevorzugt Ubbelohde-Viskosimeter], erfolgen.

Durch die zielgerichtete Einstellung der Viskosität wird ermöglicht, dass die erfindungsgemäße Zusammensetzung hervorragende Eigenschaften im Zusammenhang mit der Handhabung besitzt, d.h. ein müheloses Einsprüchen in die Nase möglich ist. Dennoch besitzt die erfindungsgemäße Zusammensetzung eine hervorragende Haftung an der Nasenschleimhaut, so dass ein Herausrinnen aus der Nase nach Applikation der Zusammensetzung verhindert wird.

Wie zuvor bereits ausgeführt, verfügt die erfindungsgemäße Zusammensetzung über eine gute Stabilität, insbesondere Lager- und Langzeitstabilität. Gemäß einer besonderen Ausführungsform ist die Zusammensetzung bei Temperaturen im Bereich von 20 °C bis 50 °C, bei einem Druck von 1.013,25 mbar (Atmosphärendruck) und bei einer relativen Luftfeuchtigkeit im Bereich von 50 % bis 90 % mindestens 6 Monate, insbesondere mindestens 12 Monate, vorzugsweise mindestens 24 Monate, bevorzugt mindestens 36 Monate, stabil, insbesondere lagerstabil.

Darüber hinaus kann es erfindungsgemäß vorgesehen sein, dass die Zusammensetzung (f) mindestens einen weiteren Inhaltsstoff, insbesondere Hilfsstoff und/oder Additiv, aufweist. Was den weiteren Hilfsstoff darüber hinaus anbelangt, so ist dieser vorzugsweise ausgewählt aus der Gruppe von Verarbeitungshilfsstoffen, Stabilisatoren, Emulgatoren, Antioxidantien, Feuchthaltemitteln, Verdickungsmitteln, Antiseptika, Farbstoffen, Aromastoffen, Riechstoffen, Duftstoffen, Streckmitteln, Bindemitteln, Netzmitteln, Vitaminen, Spurenelementen, Mineralstoffen, Mikronährstoffen und/oder ätherischen Ölen sowie deren Kombinationen.

Wie eingangs dargelegt, verfügt die erfindungsgemäße Zusammensetzung über ein breites Anwendungsprofil. Insbesondere betrifft die vorliegende Erfindung eine zuvor geschilderte Zusammensetzung zur Verwendung bei der prophylaktischen und/oder kurativen topischen Behandlung von Rhinitiden, insbesondere von *Rhinitis acuta, Rhinitis sicca, Rhinitis allergica, Rhinitis atrophicans, Rhinitis hyperplastica* oder *hypertrophicans*, *Rhinitis mutilans*, *Rhinitis vasomotorica*, umweltbedingter Rhinitis, *Rhinitis pseudomembranacea* oder *Rhinitis medicamentosa*, insbesondere *Rhinitis acuta*, *Rhinitis sicca* und/oder *Rhinitis allergica.*

Des Weiteren betrifft die vorliegende Erfindung - gemäß einem **zweiten** Aspekt - eine Applikationsvorrichtung, welche eine wie zuvor beschriebene Zusammensetzung nach der vorliegenden Erfindung enthält, wobei die erfindungsgemäße Applikationsvorrichtung insbesondere für die topische, insbesondere nasale, vorzugsweise intranasale Applikation geeignet ist und bevorzugt in Form eines Behältnisses mit Tropf- oder Sprüheinrichtung vorliegt.

Erfindungsgemäß bevorzugt ist es, wenn die erfindungsgemäße Applikationsvorrichtung eine Sprüheinrichtung zur gleichförmigen Ausbringung der Zusammensetzung in einer Menge pro Sprühstoß im Bereich von 25 µl bis 300 µl, insbesondere im Bereich von 50 µl bis 200 µl, vorzugsweise im Bereich von 75 µl bis 125 µl, aufweist.

Dabei kann es auch vorgesehen sein, dass die erfindungsgemäße Applikationsvorrichtung einen Vorratsbehälter mit einem Volumen im Bereich von 5 ml bis 100 ml, insbesondere 10 ml bis 50 ml, aufweist.

Für weitergehende Einzelheiten zu der erfindungsgemäßen Applikationsvorrichtung kann auf die vorangehenden Ausführungen zu der erfindungsgemäßen Zusammensetzung verwiesen werden, welche auch im Hinblick auf die erfindungsgemäße Applikationsvorrichtung entsprechend gelten.

Die zuvor beschriebene Zusammensetzung nach der vorliegenden Erfindung eignet sich für die Verwendung zur prophylaktischen und/oder kurativen topischen Behandlung von Rhinitiden, insbesondere von *Rhinitis acuta*, *Rhinitis sicca*, *Rhinitis allergica*, *Rhinitis atrophicans*, *Rhinitis hyperplastica* oder *hypertrophicans*, *Rhinitis mutilans*, *Rhinitis vasomotorica*, umweltbedingter Rhinitis, *Rhinitis pseudomembranacea* oder *Rhinitis medicamentosa,* insbesondere *Rhinitis acuta, Rhinitis sicca* und/oder *Rhinitis allergica.*

Gleichermaßen eignet sich die zuvor beschriebene Zusammensetzung gemäß der vorliegenden Erfindung zur Herstellung eines Arzneimittels zur prophylaktischen und/oder kurativen topischen Behandlung von Rhinitiden, insbesondere von *Rhinitis acuta, Rhinitis sicca, Rhinitis allergica, Rhinitis atrophicans, Rhinitis hyperplasti*ca oder *hypertrophicans, Rhinitis mutilans, Rhinitis vasomotorica,* umweltbedingter Rhinitis, *Rhinitis pseudomembranacea* oder *Rhinitis medicamentosa,* insbesondere *Rhinitis acuta, Rhinitis sicca* und/oder *Rhinitis allergica.*

Der Begriff des Arzneimittels (synonym auch Pharmazeutikum), wie er im Rahmen der vorliegenden Erfindung verwendet wird, ist sehr umfänglich zu verstehen und umfasst nicht nur Hilfsmittel bzw. Pharmazeutika als solche, d. h. in arzneimittelrechtlicher Hinsicht, sondern vor allem auch sogenannte Medizinprodukte und darüber hinaus auch Homöopathika und Nahrungsergänzungsmittel sowie Kosmetika und Gebrauchsgegenstände. Mit anderen Worten kann also die erfindungsgemäße Zusammensetzung in Form eines Arzneimittels (Pharmazeutikums), Medizinprodukts, Homöopathikums, Nahrungsergänzungsmittels, Kosmetikums oder Gebrauchsgegenstands vorliegen.

Insbesondere kann die erfindungsgemäße Zusammensetzung zur prophylaktischen und/oder kurativen topischen Behandlung von Rhinitiden, insbesondere von *Rhinitis acuta*, *Rhinitis sicca*, *Rhinitis allergica*, *Rhinitis atrophicans*, *Rhinitis hyperplasti*ca oder *hypertrophicans*, *Rhinitis mutilans*, *Rhinitis vasomotorica*, umweltbedingter Rhinitis, *Rhinitis pseudomembranacea* oder *Rhinitis medicamentosa*, insbesondere *Rhinitis acuta*, *Rhinitis sicca* und/oder *Rhinitis allergica*, verwendet werden.

Wie zuvor beschrieben, kann im Rahmen der Verwendung die erfindungsgemäße Zusammensetzung, wie zuvor beschrieben, topisch, insbesondere nasal, vorzugsweise intranasal, appliziert werden. Dabei kann insbesondere die erfindungsgemäße Applikationsvorrichtung, wie sie zuvor beschrieben worden ist, zur Anwendung kommen.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die folgenden Ausführungsbeispiele dienen lediglich der Veranschaulichung der vorliegenden Erfindung, ohne die vorliegende Erfindung jedoch hierauf zu beschränken.

### AUSFÜHRUNGSBEISPIELE

### 1. Herstellungsbeispiele

### Allgemeine Herstellvorschrift

Zur Herstellung von jeweils 1.000 g einer klaren wässrigen Lösung der erfindungsgemäßen Zusammensetzung wird in dem Fachmann an sich bekannter Weise vorgegangen:
Zunächst wird bei Raumtemperatur und Umgebungsdruck in einem Glasgefäß mit Rühreinrichtung eine definierte Menge an gereinigtem Wasser vorgelegt und nachfolgend mit einer Lösung des gewählten Puffersystems auf einen vorgegebenen pH-Wert im Bereich 5,25 bis 5,85 (z. B. pH-Wert von ca. 5,5) unter anschließender Kontrolle des erreichten pH-Werts eingestellt. Anschließend werden hierzu die nachfolgenden in den Rezepturbeispielen spezifizierten Mengen der weiteren Wirk- und Inhaltsstoffe (Dexpanthenol, Hydroxypropylmethylcellulose, Osmolyte sowie gegebenenfalls Konservierungs-/Desinfektionsmittel etc.) hinzugegeben. Die Mischung wird durch gründliches Rühren klar gelöst. Nachfolgend wird die Mischung mit weiterem Wasser zum Endgewicht von 1.000 g aufgefüllt und homogen gerührt. Gegebenenfalls wird die Lösung über neutrale Filter filtriert. Abschließend wird der pH-Wert noch einmal bestimmt. Auch die übrigen entsprechenden Spezifikationen der Lösungen werden auf die eingestellten bzw. vorgewählten Wertebereiche hin überprüft (z. B. Osmolalität; relative Dichte; mikrobiologische Reinheit und Sterilität; Ausschluss von Verunreinigungen, insbesondere Abbauprodukten der Inhalts- und Wirkstoffe; Viskosität; Aussehen). Ein Teil der erhaltenen Lösung wird anschließend in Enghalsflaschen aus Braunglas zu 10 ml oder 20 ml abgefüllt, welche wahlweise mit einer Tropfpipette oder einer Sprühdosierpumpe ausgerüstet werden können; zur bestimmungsgemäßen Anwendung können mehrmals täglich 1 bis 3 Tropfen bzw. 1 bis 2 Sprühstöße in jedes Nasenloch gegeben und aufgezogen werden. Ein anderer Teil der erhaltenen Lösungen wird für Stabilitätsuntersuchungen verwendet.

Nach dieser allgemeinen Herstellvorschrift werden die nachfolgend spezifizierten Rezepturen hergestellt.

**Rezeptur 1 (Angabe pro 1.000 g Zusammensetzung)**

| **Inhaltsstoff** | **Menge / Gew.-%** | **Qualität** |
|---|---|---|
| Dexpanthenol | 0,5 | Ph. Eur. |
| Hydroxypropylmethylcellulose (HPMC) | 0,02 | Ph. Eur. |
| Puffersystem: | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Kaliumsorbat) | 0,2 | Ph. Eur. |
| Osmolyt (Natriumchlorid) | 2,5 | Ph. Eur. |
| Gereinigtes Wasser | 95,9 | Ph. Eur. |

**Rezeptur 2 (Angabe pro 1.000 g Zusammensetzung)**

| **Inhaltsstoff** | **Menge / Gew.-%** | **Qualität** |
|---|---|---|
| Dexpanthenol | 0,5 | Ph. Eur. |
| HPMC | 0,001 | Ph. Eur. |
| Puffersystem : | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Kaliumsorbat) | 0,2 | Ph. Eur. |
| Osmolyt (Natriumchlorid) | 2,5 | Ph. Eur. |
| Gereinigtes Wasser | 95,919 | Ph. Eur. |

**Rezeptur 3 (Angabe pro 1.000 g Zusammensetzung)**

| **Inhaltsstoff** | **Menge / Gew.-%** | **Qualität** |
|---|---|---|
| Dexpanthenol | 0,5 | Ph. Eur. |
| HPMC | 1,0 | Ph. Eur. |
| Puffersystem: | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Kaliumsorbat) | 0,2 | Ph. Eur. |
| Osmolyt (Natriumchlorid) | 2,5 | Ph. Eur. |
| Gereinigtes Wasser | 94,92 | Ph. Eur. |

**R zeptur 4 (Angabe pro 1.000 g Zusammensetzung)**

| **Inhaltsstoff** | **Menge / Gew.-%** | **Qualität** |
|---|---|---|
| Dexpanthenol | 0,5 | Ph. Eur. |
| HPMC | ---- | Ph. Eur. |
| Puffersystem : | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Kaliumsorbat) | 0,2 | Ph. Eur. |
| Osmolyt (Natriumchlorid) | 2,5 | Ph. Eur. |
| Gereinigtes Wasser | 95,92 | Ph. Eur. |

**Rezeptur 5 (Angabe pro 1.000 g Zusammensetzung)**

| **Inhaltsstoff** | **Menge / Gew.-%** | **Qualität** |
|---|---|---|
| Dexpanthenol | ---- | Ph. Eur. |
| HPMC | 0,02 | Ph. Eur. |
| Puffersystem: | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Kaliumsorbat) | 0,2 | Ph. Eur. |
| Osmolyt (Natriumchlorid) | 2,5 | Ph. Eur. |
| Gereinigtes Wasser | 96,4 | Ph. Eur. |

**Rezeptur 6 (Angabe pro 1.000 g Zusammensetzung)**

| **Inhaltsstoff** | **Menge / Gew.-%** | **Qualität** |
|---|---|---|
| Dexpanthenol | 0,5 | Ph. Eur. |
| HPMC | 0,02 | Ph. Eur. |
| Puffersystem: | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Benzalkoniumchlorid) | 0,2 | Ph. Eur. |
| Osmolyt (Natriumchlorid) | 2,5 | Ph. Eur. |
| Gereinigtes Wasser | 95,9 | Ph. Eur. |

**Rezeptur 7 (Angabe pro 1.000 g Zusammensetzung)**

| **Inhaltsstoff** | **Menge / Gew.-%** | **Qualität** |
|---|---|---|
| Dexpanthenol | 0,5 | Ph. Eur. |
| HPMC | 0,02 | Ph. Eur. |
| Puffersystem: | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) (Benzylalkohol) | 0,2 | Ph. Eur. |
| Osmolyt (Natriumchlorid) | 2,5 | Ph. Eur. |
| Gereinigtes Wasser | 95,9 | Ph. Eur. |

**Rezeptur 8 (Angabe pro 1.000 g Zusammensetzung)**

| **Inhaltsstoff** | **Menge / Gew.-%** | **Qualität** |
|---|---|---|
| Dexpanthenol | 0,5 | Ph. Eur. |
| HPMC | 0,02 | Ph. Eur. |
| Puffersystem: | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) | ---- | Ph. Eur. |
| Osmolyt (Natriumchlorid) | 2,5 | Ph. Eur. |
| Gereinigtes Wasser | 96,1 | Ph. Eur. |

**Rezeptur 9 (Angabe pro 1.000 g Zusammensetzung)**

| **Inhaltsstoff** | **Menge / Gew.-%** | **Qualität** |
|---|---|---|
| Dexpanthenol | ---- | Ph. Eur. |
| HPMC | ---- | Ph. Eur. |
| Puffersystem: | | Ph. Eur. |
| Kaliumdihydrogenphosphat / | 0,853 | |
| Natriummonohydrogenphosphat (Dodekahydrat) | 0,027 | |
| Konservierungs-/Desinfektionsmittel (Antiseptikum) | ---- | Ph. Eur. |
| Osmolyt (Natriumchlorid) | ---- | Ph. Eur. |
| Gereinigtes Wasser | 99,12 | Ph. Eur. |

### 2. Stabilitätsuntersuchungen

### a) Stabilitätsverhalten in Abhängigkeit von dem eingesetzten Konservierungs- bzw. Desinfektionsmittel

Entsprechend der vorstehenden allgemeinen Herstellvorschrift werden wässrige Zusammensetzungen gemäß den Rezepturen 1 sowie 6 bis 8 hergestellt (Zusammensetzungen A1 sowie A6 bis A8). Die Zusammensetzungen weisen jeweils 0,5 Gew.-% Dexpanthenol, 0,02 Gew.-% Hydroxypropylmethylcellulose sowie 2,5 Gew.-% Natriumchlorid als Osmolyt auf. Mit einem wie zuvor spezifizierten pH-Wert Puffersystem auf Phosphatbasis wird jeweils ein pH-Wert im Bereich von 5,25 bis 5,85 eingestellt (0,853 Gew.-% Kaliumdihydrogenphosphat und 0,027 Gew.-% Natriummonohydrogenphosphat-Dodekahydrat, jeweils bezogen auf die Zusammensetzung).

Die erfindungsgemäße Zusammensetzung A1 gemäß Rezeptur 1 enthält zudem als Konservierungs- bzw. Desinfektionsmittel 0,2 Gew.-% Kaliumsorbat. Die Zusammensetzung A6 gemäß Rezeptur 6 enthält als Konservierungs- bzw. Desinfektionsmittel 0,2 Gew.-% Benzalkoniumchlorid. Die Zusammensetzung A7 gemäß Rezeptur 7 enthält als Konservierungs- bzw. Desinfektionsmittel 0,2 Gew.-% Benzylalkohol. Die Zusammensetzung A8 gemäß Rezeptur 8 enthält kein Konservierungs- bzw. Desinfektionsmittel.

Das Stabilitätsverhalten dieser Zusammensetzungen wird unter definierten Bedingungen untersucht (Lagerung jeweils bei 25 °C, bei einem Druck von 1.013,25 mbar [Atmosphärendruck] und bei einer relativen Luftfeuchtigkeit von 75 %). Die nachfolgenden Angaben beziehen sich auf die maximale Lagerfähigkeit (Anforderungen an die Stabilität: Abbau Dexpanthenol ≤ 1%, bezogen auf Dexpanthenol; Abbau Hydroxypropylmethylcellulose ≤ 1%, bezogen auf Hydroxypropylmethylcellulose):

| Zusammensetzung | Lagerfähigkeit |
|---|---|
| A1 | 45,5 Monate |
| A6 | 44,5 Monate |
| A7 | 39,5 Monate |
| A8 | 37,5 Monate |

Wie die Untersuchungen zeigen, werden besonders gute Lagerfähigkeiten erhalten, wenn die Zusammensetzungen als Konservierungsmittel Kaliumsorbat enthalten. Darüber hinaus werden auch gute Ergebnisse erzielt, wenn als Konservierungsmittel Benzalkoniumchlorid eingesetzt wird. Keine zufriedenstellende Lagerstabilität hingegen wird erzielt, wenn keinerlei Konservierungsmittel eingesetzt wird.

### b) Untersuchungen zum antibakteriellen und antiviralen Wirkverhalten

Die in dem vorstehenden Ausführungsbeispiel 2a) beschriebenen Zusammensetzungen A1 sowie A6 bis A8 werden auf ihr antibakterielles bzw. antivirales Wirkverhalten getestet.

Die antibakterielle Aktivität der Zusammensetzung wird in einem Plattendiffusionstest sowie in einer Verdünnungsreihe an 18 Bakterienstämmen (hierunter *Staphylococcus aureus*, *Haemophilus influenzae*, *Moraxella catarrhalis*, *Streptococcus pneumoniae*, *Bacillus sp*., *Citrobacter sp*., *Escherichia coli*, *Klebsiella sp*., *Salmonella sp.* und *Vibrio cholerae*) getestet (Ermittlungen der MIC-Konzentrationen [*Minimal Inhibitory Concentrations* bzw. minimale Hemmkonzentration]). Die Zusammensetzungen A1, A6 und A7 zeigen eine gute antibakterielle Aktivität, wobei die Zusammensetzung A1 die beste antibakterielle Aktivität zeigt, gefolgt von den Zusammensetzungen A6 und A7. Zusammensetzung A8 hingegen zeigt im Wesentlichen keine antibakterielle Aktivität.

Die antivirale Aktivität der Zusammensetzungen wird gegen IBV (Infektiöses Bronchitis-Virus, IBV, Gray Stamm in einem sogenannten MTT-Test in Vero-Zellen (*African Green Monkey Kidney Cells* [Affennierenzellen]) getestet (Ermittlungen der IC₅₀-Konzentrationen). Die Zusammensetzungen A1 sowie A6 und A7 zeigen eine gute antivirale Aktivität (Anti-IBV-Eigenschaften), wobei die Zusammensetzung A1 die beste antivirale Aktivität zeigt, gefolgt von den Zusammensetzungen A6 und A7 (in der Reihenfolge der antiviralen Wirksamkeit). Die Zusammensetzung A8 zeigt im Wesentlichen keine antivirale Aktivität.

### 3. Anwendungsbeobachtungen und klinische Untersuchungen

Entsprechend der vorstehenden allgemeinen Herstellvorschrift werden wässrige Zusammensetzungen A1 bis A5 sowie eine Zusammensetzung A9 entsprechend den Rezepturen 1 bis 5 bzw. entsprechend der Rezeptur 9 hergestellt. Die Zusammensetzungen A1 bis A5 und A9 weisen ein wie zuvor spezifiziertes pH-Wert-Puffersystem auf Phosphatbasis auf, wobei der pH-Wert jeweils auf einen Wert im Bereich von 5,25 bis 5,85 eingestellt ist (0,85 Teil-Gew.-% Kaliumdihydrogenphosphat und 0,027 Gew.-% Natriummonohydrogenphosphat-Dodekahydrat, jeweils bezogen auf die Zusammensetzung).

Die erste erfindungsgemäße Zusammensetzung A1 weist als Inhaltsstoffe 0,5 Gew.-% Dexpanthenol und darüber hinaus 0,02 Gew.-% Hydroxypropylmethylcellulose auf. Die Zusammensetzung A2 weist ebenfalls 0,5 Gew.-% Dexpanthenol, jedoch nur 0,01 Gew.-% Hydroxypropylmethylcellulose auf. Zusammensetzung A3 enthält 0,5 Gew.-% Dexpanthenol und 1 Gew.-% Hydroxypropylmethylcellulose. Die Zusammensetzung A4 enthält als Wirkstoff lediglich 0,5 Gew.-% Dexpanthenol. Die Zusammensetzung A5 enthält als Wirkstoff kein Dexpanthenol und lediglich 0,02 Gew.-% Hydroxypropylmethylcellulose. Die vorgenannten Zusammensetzungen A1 bis A5 sind zudem als hypertone Lösungen ausgebildet und enthalten 2,5 Gew.-% Natriumchlorid als Osmolyt.

Die Zusammensetzung A9 dient als Placebo und weist als Inhaltsstoff lediglich das erfindungsgemäß verwendete Puffersystem und gereinigtes Wasser, jedoch keine der übrigen erfindungsgemäß eingesetzten Inhaltsstoffe auf (Dexpanthenol, HPCM, Natriumchlorid, Konservierungsmittel).

Die zuvor geschilderten Zusammensetzungen A1 bis A5 und A9 werden in klinischen Anwendungen (randomisierte Placebo-kontrollierte Doppelblindstudie) getestet.

Je 12 Probanden eines aus 72 Patienten bestehenden Probanden-Kollektivs im Alter von 18 von 70 Jahren (35 männlich, 37 weiblich) jeweils mit akuten viralen Rhinitiden (Erkältungsschnupfen) werden während der Dauer ihrer Erkrankung 5 Tage lang mit den Zusammensetzungen A1 bis A5 behandelt, wobei die Zusammensetzungen A1 bis A5 mehrmals täglich als Spray topisch appliziert werden. Weitere 12 Probanden des Probanden-Kollektivs werden mit einer nicht erfindungsgemäßen Zusammensetzung A9 therapiert, welche keinerlei Wirkstoffe enthält (Placebo-Kontrolle).

Als Zielvariablen werden die Bewertung der Nasenatmungsbehinderung, der Rhinorrhö (Fließschnupfen), der Rötung der Nasenschleimhaut, der Trockenheit der Nasenschleimhaut, der Borkenbildung, des Heilungsverlaufs und der Verträglichkeit dokumentiert.

Alle getesteten Zusammensetzungen zeigen eine hervorragende Verträglichkeit. Im Zusammenhang mit einer Beschleunigung des Heilungsverlaufs wird mit den Zusammensetzungen A1 und A3 das beste Ergebnis erzielt, gefolgt von Zusammensetzung A2. Mit den Zusammensetzungen A4 und A5 sowie der Placebo-Kontrolle A9 hingegen werden keine zufriedenstellenden Ergebnisse erzielt. Mit Zusammensetzung A2, welche Hydroxypropylmethylcellulose nur in sehr geringen Mengen enthält, wird nur eine leichte Beschleunigung des Heilungsverlaufs erzielt, welche jedoch immer noch die Ergebnisse der Zusammensetzungen A4 und A5 sowie der Placebo-Kontrolle A9 übertrifft.

Bezüglich der übrigen Zielvariablen, d. h Bewertung der Nasenatmungsbehinderung, Bewertung der Rhinorrhö (Fließschnupfen) und Bewertung der Rötung der Nasenschleimhaut, sind die Bewertungen in Form der sogenannten Scores (Score-Werte) in der nachfolgenden Tabelle für die verschiedenen Zusammensetzungen wiedergegeben (0 = keine, 1 = geringe, 2 = mäßige, 3 = starke und 4 = sehr starke Ausprägung); ein höherer Score-Wert zeigt somit eine höhere Aktivität der Erkrankung bzw. eine schlechtere Wirksamkeit an.

| | **Zusammensetzung** | | | | | |
|---|---|---|---|---|---|---|
| | **A1** | **A2** | **A3** | **A4*** | **A5*** | **A9*** |
| Nasenatmungs-Behinderung | | | | | | |
| Basiswert | 3,1±0,3 | 2,8±0,6 | 2,8±0,2 | 3,2±0,4 | 3,0±0,5 | 2,9±0,3 |
| 3 Tage | 1,8±0,6 | 2,1±0,3 | 1,8±0,3 | 2,5±0,5 | 2,8±0,3 | 2,7±0,7 |
| 5 Tage | 1,0±0,5 | 1,8±0,6 | 1,1±0,4 | 2,2±0,2 | 2,3±0,6 | 2,6±0,2 |
| | | | | | | |

| Rhinorrhö | | | | | | |
|---|---|---|---|---|---|---|
| Basiswert | 2,8±0,2 | 3,2±0,4 | 3,1±0,6 | 2,9±0,3 | 3,0±0,2 | 2,9±0,3 |
| 3 Tage | 1,7±0,7 | 2,9±0,3 | 1,6±0,5 | 2,6±0,7 | 2,7±0,8 | 2,8±0,2 |
| 5 Tage | 1,0±0,2 | 2,2±0,4 | 1,2±0,3 | 2,4±0,6 | 2,3±0,1 | 2,6±0,3 |
| | | | | | | |

| Rötung der Nasenschleimhaut | | | | | | |
|---|---|---|---|---|---|---|
| Basiswert | 3,1±0,6 | 3,2±0,5 | 3,0±0,3 | 2,9±0,6 | 3,0±0,3 | 3,2±0,6 |
| 3 Tage | 1,3±0,2 | 2,8±0,7 | 1,6±0,7 | 2,8±0,7 | 2,9±0,7 | 3,0±0,3 |
| 5 Tage | 0,9±0,5 | 2,2±0,6 | 1,2±0,3 | 2,6±0,3 | 2,5±0,3 | 2,9±0,7 |

| | | | | | | |
|---|---|---|---|---|---|---|
| * nicht erfindungsgemäß | | | | | | |

Insgesamt zeigen die getesteten erfindungsgemäßen Zusammensetzungen A1 bis A3 nach der vorliegenden Erfindung eine gute Wirksamkeit bei der Behandlung akuter Rhinitiden. Bezüglich der Wirksamkeit liefern die erfindungsgemäßen Zusammensetzungen A1 und A3 das beste Ergebnis, gefolgt von der Zusammensetzung A2. Die nicht erfindungsgemäßen Zusammensetzungen A4 und A5 sowie die Placebo-Kontrolle A9 hingegen zeigen keine zufriedenstellende bzw. zumindest im Wesentlichen keine Wirksamkeit.

Weitergehende klinische Untersuchungen sowie weiterführende randomisierte Placebo-kontrollierte Doppelblindstudien bestätigen darüber hinaus einen Synergismus der Wirkungen der Wirkstoffe (d. h. Dexpanthenol einerseits sowie Hydroxypropylmethylcellulose andererseits insbesondere in hypertoner Lösung) der erfindungsgemäßen Zusammensetzungen, was bei der Behandlung von Rhinitiden zu einer klinisch deutlicheren bzw. signifikanteren Besserung führt, und zwar über das Ausmaß der Einzelwirkstoffe hinausgehend. Was die eingesetzte Menge von Hydroxypropylmethylcellulose jedoch anbelangt, so lässt sich durch den Einsatz größerer Mengen die Wirksamkeit nicht signifikant steigern, so dass mit einer Einsatzmenge von 0,02 Gew.-% eine optimale Balance zwischen hervorragender Wirksamkeit einerseits sowie guter Handhabung (gute Sprühapplikation ohne Herausrinnen aus der Nase) andererseits geschaffen wird.

## Patentansprüche

1. Zusammensetzung zur Verwendung bei der topischen nasalen, bevorzugt intranasalen Applikation für die Behandlung von Rhinitiden,
wobei die Zusammensetzung in Kombination und jeweils in wirksamen, insbesondere pharmazeutisch wirksamen Mengen enthält:
(a) Pantothenol, vorzugsweise Dexpanthenol (D-Pantothenol), oder dessen physiologisch unbedenkliche Ester und/oder Pantothensäure oder deren physiologisch unbedenkliche Salze (Komponente (a)), wobei die Zusammensetzung die Komponente (a) in einer Menge im Bereich von 0,001 bis 20 Gew.-%, bezogen auf die Zusammensetzung, enthält; und
(b) Cellulose und/oder ein physiologisch unbedenkliches Cellulosederivat aus der Gruppe von Carboxymethylcellulose, Methylcellulose, Ethylcellulose, Hydroxyethylcellulose, Hydroxyethylmethylcellulose und/oder Hydroxypropylmethylcellulose (Komponente (b)), wobei die Zusammensetzung die Komponente (b) in einer Menge im Bereich von 0,0001 bis 5 Gew.-%, bezogen auf die Zusammensetzung, enthält;
(c) mindestens ein Konservierungsmittel (Komponente (c)), wobei die Zusammensetzung die Komponente (c) in einer Menge im Bereich von 0,001 bis 10 Gew.-%, bezogen auf die Zusammensetzung, enthält und wobei die Komponente (c) ausgewählt ist aus der Gruppe von Sorbinsäure und/oder deren pharmazeutisch verträglichen Salzen (Sorbaten);
wobei die Zusammensetzung gegenüber den Zellen der Nasenschleimhaut hyperton ausgebildet ist.

2. Zusammensetzung zur Verwendung nach Anspruch 1,
wobei die Zusammensetzung die Komponente (a) in Form von Dexpanthenol (D-Pantothenol) oder dessen physiologisch unbedenklichen Estern, vorzugsweise Dexpanthenol (D-Pantothenol), enthält; und/oder
wobei die Zusammensetzung die Komponente (a) in einer Menge im Bereich von 0,005 bis 15 Gew.-%, vorzugsweise 0,01 bis 10 Gew.-%, bevorzugt 0,1 bis 1 Gew.-%, besonders bevorzugt 0,2 bis 0,8 Gew.-%, bezogen auf die Zusammensetzung, enthält; und/oder
wobei das Cellulosederivat Hydroxypropylmethylcellulose ist; und/oder wobei die Zusammensetzung die Komponente (b) in einer Menge im Bereich von 0,0001 bis 2 Gew.-%, vorzugsweise 0,001 bis 1,5 Gew.-%, bevorzugt 0,005 bis 1 Gew.-%, besonders bevorzugt 0,01 bis 0,1 Gew.-%, noch mehr bevorzugt 0,02 bis 0,05 Gew.-%, bezogen auf die Zusammensetzung, enthält; und/oder wobei die Zusammensetzung die Komponente (a) und die Komponente (b) in einem gewichtsbezogen Verhältnis von (a) : (b) im Bereich von 1 : 1 bis 500 : 1, insbesondere 1,25 : 1 bis 100 : 1, vorzugsweise 1,5 : 1 bis 75 : 1, bevorzugt 2 : 1 bis 50 : 1, besonders bevorzugt 10 : 1 bis 30 : 1, enthält.

3. Zusammensetzung zur Verwendung nach einem der vorangehenden Ansprüche,
wobei die Komponente (c) ein Alkali- oder Erdalkalisorbat, vorzugsweise Alkalisorbat, bevorzugt Kaliumsorbat, ist, und/oder
wobei die Zusammensetzung die Komponente (c) in einer Menge im Bereich von 0,005 bis 5 Gew.-%, vorzugsweise im Bereich 0,01 bis 1 Gew.-%, bevorzugt im Bereich von 0,1 bis 0,5 Gew.-%, bezogen auf die Zusammensetzung, enthält, und/oder
wobei die Zusammensetzung die Komponente (a) und die Kompo-nente (c) in einem gewichtsbezogen Verhältnis von (a) : (c) im Bereich von 250 : 1 bis 1 : 10, insbesondere 100 : 1 bis 1 : 1, vorzugsweise 50 : 1 bis 1 : 1, bevorzugt 25 : 1 bis 1,25 : 1, besonders bevorzugt 10 : 1 bis 2 : 1, ganz besonders bevorzugt 5 : 1 bis 2 : 1, enthält, und/oder
wobei die Zusammensetzung die Komponente (b) und die Komponente (c) in einem gewichtsbezogen Verhältnis von (b) : (c) im Bereich von 1 : 500 bis 10 : 1, insbesondere 1 : 250 bis 5 : 1, vorzugsweise 1 : 200 bis 2 : 1, bevorzugt 1 : 50 bis 1 : 1, besonders bevorzugt 1 : 20 bis 1 : 2, noch mehr bevorzugt 1 : 15 bis 1 : 5, enthält.

4. Zusammensetzung zur Verwendung nach einem der vorangehenden Ansprüche,
wobei die Zusammensetzung (d) mindestens ein chemisches Puffersystem, insbesondere in Form von Puffersalz(en), enthält,
insbesondere wobei die Zusammensetzung das chemische Puffersystem, bezogen auf die Zusammensetzung und berechnet als Summe aller Bestandteile des chemischen Puffersystems, in einer Menge im Bereich von 0,001 bis 4 Gew.-%, insbesondere 0,01 bis 3 Gew.-%, vorzugsweise 0,05 bis 2 Gew.-%, bevorzugt 0,1 bis 1,5 Gew.-%, besonders bevorzugt 0,2 bis 1 Gew.-%, enthält, und/oder
insbesondere wobei das chemische Puffersystem zur Einstellung und/oder Konstanthaltung des pH-Werts der Zusammensetzung dient, und/oder insbesondere wobei das chemische Puffersystem als ein Dihydrogenphosphat/Monohydrogenposphat-Puffersystem ("H₂PO₄⁻/HPO₄²⁻-Puffer(system)" bzw. "Phosphatpuffer(system)"), insbesondere als ein Alkalidihydrogenphosphat/Alkalimonohydrogenposphat-Puffersystem, vorliegt, bevorzugt mit einem Dihydrogenphosphat/Monohydrogenposphat-Molverhältnis von größer 5:1, insbesondere im Bereich von 5 : 1 bis 200 : 1, vorzugsweise 6 : 1 bis 175 : 1, bevorzugt 7 : 1 bis 150 : 1, besonders bevorzugt 8 : 1 bis 125 : 1, ganz besonders bevorzugt 10 : 1 bis 100 : 1, noch mehr bevorzugt 20 : 1 bis 90 : 1, und/oder
insbesondere wobei die Zusammensetzung die Komponente (a) und die Komponente (d) in einem gewichtsbezogen Verhältnis von (a) : (d) im Bereich von 1 : 100 bis 100 : 1, insbesondere 1 : 50 bis 50 : 1, vorzugsweise 1 : 20 bis 20 : 1, bevorzugt 1 : 10 bis 10: 1, besonders bevorzugt 1 : 5 bis 2 : 1, ganz besonders bevorzugt 1 : 2 bis 1 : 1, enthält, und/oder
insbesondere wobei die Zusammensetzung die Komponente (b) und die Komponente (d) in einem gewichtsbezogen Verhältnis von (b) : (d) im Bereich von 1 : 1.000 bis 100 : 1, insbesondere 1 : 500 bis 10 : 1, vorzugsweise 1 : 200 bis 1 : 1, bevorzugt 1 : 150 bis 1 : 15, vorzugsweise 1 : 100 bis 1 : 30, enthält, und/oder
insbesondere wobei die Zusammensetzung die Komponente (c) und die Komponente (d) in einem gewichtsbezogen Verhältnis von (c) : (d) im Bereich von 1 : 500 bis 100 : 1, insbesondere 1 : 100 bis 5 : 1, vorzugsweise 1 : 20 bis 3 : 1, bevorzugt 1 : 10 bis 1 : 1, besonders bevorzugt 1 : 6 bis 1 : 2, enthält.

5. Zusammensetzung zur Verwendung nach einem der vorangehenden Ansprüche,
wobei die Zusammensetzung einen pH-Wert im Bereich von 4,5 bis 8,0, insbesondere im Bereich von 5,0 bis 6,5, vorzugsweise im Bereich von 5,0 bis 6,2, bevorzugt im Bereich von 5,0 bis 6,0, noch mehr bevorzugt im Bereich von 5,1 bis 6,0, ganz besonders bevorzugt im Bereich von 5,2 bis 5,9, am meisten bevorzugt im Bereich von 5,25 bis 5,85, aufweist und/oder wobei der pH-Wert der Zusammensetzung Wert im Bereich von 4,5 bis 8,0, insbesondere im Bereich von 5,0 bis 6,5, vorzugsweise im Bereich von 5,0 bis 6,2, bevorzugt im Bereich von 5,0 bis 6,0, noch mehr bevorzugt im Bereich von 5,1 bis 6,0, ganz besonders bevorzugt im Bereich von 5,2 bis 5,9, am meisten bevorzugt im Bereich von 5,25 bis 5,85, eingestellt und/oder konstant gehalten wird, vorzugsweise mittels mindestens eines chemischen Puffersystems; und/oder
wobei die Zusammensetzung (e) mindestens einen physiologisch unbedenklichen bzw. verträglichen, osmotisch aktiven Stoff und/oder mindestens einen physiologisch unbedenklichen bzw. verträglichen Osmolyten enthält,
insbesondere wobei (e) die osmotisch aktiven Stoffe und/oder die Osmolyte ausgewählt sind aus der Gruppe von Ionen von physiologisch unbedenklichen bzw. verträglichen Salzen (d.h. Salz-Ionen), insbesondere Alkali-Ionen, insbesondere Natrium-Ionen und/oder Kalium-Ionen, und/oder Chlorid-Ionen, vorzugsweise Natrium-Ionen und Chlorid-Ionen; Zuckern, insbesondere Trehalose und/oder Saccharose; Polyolen, insbesondere Glycerin; und/oder Aminosäuren, insbesondere Glutaminsäure und/oder dessen Salzen, Prolin, (Hydroxy-)Ectoin und/oder Glycinbetain, und/oder
insbesondere wobei die osmotisch aktiven Stoffe und/oder die Osmolyte Natrium-Ionen und Chlorid-Ionen, insbesondere in Form von Natriumchlorid, sind; und/oder wobei die Zusammensetzung (e) physiologisch verträgliche, osmotisch aktive Stoffe und/oder physiologisch verträgliche Osmolyte in einer Menge im Bereich von 0,1 bis 25 Gew.-%, insbesondere 1 bis 20 Gew.-%, vorzugsweise 1 bis 15 Gew.-%, bevorzugt 1,5 bis 10 Gew.-%, besonders bevorzugt 2 bis 5 Gew.-%, bezogen auf die Zusammensetzung, enthält.

6. Zusammensetzung zur Verwendung nach einem der vorangehenden Ansprüche,
wobei die Zusammensetzung in Kombination und jeweils in wirksamen, insbesondere pharmazeutisch wirksamen Mengen enthält:
(a) Pantothenol, vorzugsweise Dexpanthenol (D-Pantothenol), oder dessen physiologisch unbedenkliche Ester und/oder Pantothensäure oder deren physiologisch unbedenkliche Salze (Komponente (a)) in einer Menge im Bereich von 0,001 bis 20 Gew.-%, insbesondere 0,005 bis 15 Gew.-%, vorzugsweise 0,01 bis 10 Gew.-%, bevorzugt 0,1 bis 1 Gew.-%, besonders bevorzugt 0,2 bis 0,8 Gew.-%, bezogen auf die Zusammensetzung; und
(b) Cellulose und/oder mindestens ein physiologisch unbedenkliches Cellulosederivat (Komponente (b)) in einer Menge im Bereich von 0,0001 bis 5 Gew.-%, insbesondere 0,0001 bis 2 Gew.-%, vorzugsweise 0,001 bis 1,5 Gew.-%, bevorzugt 0,005 bis 1 Gew.-%, besonders bevorzugt 0,01 bis 0,1 Gew.-%, noch mehr bevorzugt 0,02 bis 0,05 Gew.-%, bezogen auf die Zusammensetzung, wobei die Komponente (b) ausgewählt ist aus physiologisch unbedenklichen Cellulosederivaten und/oder modifizierten Cellulosen in Form von Celluloseethern und/oder Celluloseestern;
(c) mindestens ein Konservierungsmittel (Komponente (c)) in einer Menge im Bereich von 0,001 bis 10 Gew.-%, insbesondere im Bereich von 0,005 bis 5 Gew.-%, vorzugsweise im Bereich 0,01 bis 1 Gew.-%, bevorzugt im Bereich von 0,1 bis 0,5 Gew.-%, bezogen auf die Zusammensetzung, wobei die Komponente (c) ausgewählt ist aus der Gruppe von Sorbinsäure und/oder deren pharmazeutisch verträglichen Salzen (Sorbaten);
(d) gegebenenfalls mindestens ein chemisches Puffersystem (Komponente (d)), insbesondere in Form von Puffersalz(en), insbesondere in einer Menge im Bereich von 0,001 bis 4 Gew.-%, insbesondere 0,01 bis 3 Gew.-%, vorzugsweise 0,05 bis 2 Gew.-%, bevorzugt 0,1 bis 1,5 Gew.-%, besonders bevorzugt 0,2 bis 1 Gew.-%, bezogen auf die Zusammensetzung und berechnet als Summe aller Bestandteile des chemischen Puffersystems; und
(e) gegebenenfalls mindestens einen physiologisch unbedenklichen bzw. verträglichen, osmotisch aktiven Stoff und/oder physiologisch unbedenklichen bzw. verträglichen Osmolyten (Komponente (e)) in einer Menge im Bereich von 0,1 bis 25 Gew.-%, insbesondere 1 bis 20 Gew.-%, vorzugsweise 1 bis 15 Gew.-%, bevorzugt 1,5 bis 10 Gew.-%, besonders bevorzugt 2 bis 5 Gew.-%, bezogen auf die Zusammensetzung.

7. Zusammensetzung zur Verwendung nach einem der vorangehenden Ansprüche,
wobei die Zusammensetzung als wässrige Zusammensetzung vorliegt und/oder wobei die Zusammensetzung wässrig basiert ist und/oder wässrig formuliert vorliegt, insbesondere in Form einer wässrigen Lösung oder wässrigen Solubilisierung; und/oder
wobei die Zusammensetzung Wasser, insbesondere gereinigtes Wasser, in einer Menge im Bereich von 60 bis 99 Gew.-%, insbesondere im Bereich von 70 bis 98 Gew.-%, bevorzugt im Bereich von 80 bis 97 Gew.-%, besonders bevorzugt im Bereich von 90 bis 96 Gew.-%, bezogen auf die Zusammensetzung, enthält und/oder wobei die Zusammensetzung Wasser als pharmazeutisch verträglichen Träger (Exzipienten) enthält und/oder wobei die Zusammensetzung wässrig basiert ausgebildet ist; und/oder
wobei die Zusammensetzung eine Osmolalität im Bereich von 300 bis 600 mosm/kg, insbesondere im Bereich von 310 bis 550 mosm/kg, vorzugsweise im Bereich von 300 bis 525 mosm/kg, bevorzugt im Bereich von 325 bis 510 mosm/kg, besonders bevorzugt im Bereich von 350 bis 500 mosm/kg, aufweist; und/oder
wobei die Zusammensetzung bei einer Temperatur von 20 °C und bei einem Druck von 1.013,25 mbar (Atmosphärendruck) eine relative Dichte, bezogen auf reines Wasser, im Bereich von 1,001 bis 1,5, insbesondere im Bereich von 1,001 bis 1,2, vorzugsweise im Bereich von 1,005 bis 1,15, bevorzugt im Bereich von 1,005 bis 1,1, aufweist.

8. Zusammensetzung zur Verwendung nach einem der vorangehenden Ansprüche,
wobei die Zusammensetzung bei einer Temperatur von 20 °C eine dynamische Viskosität von mindestens 1,1 mPas, insbesondere im Bereich von 1,1 bis 10⁵ mPas, vorzugsweise im Bereich von 1,2 bis 10⁴ mPas, besonders bevorzugt im Bereich von 1,3 bis 10³ mPas, noch mehr bevorzugt im Bereich von 1,5 bis 100 mPas, aufweist (insbesondere bestimmt gemäß der Methode nach Ph.Eur. 7. Ausgabe, Grundwerk 2011, Abschnitt 2.2.9 "Kapillarviskosimeter"); und/oder
wobei die Zusammensetzung bei Temperaturen im Bereich von 20 °C bis 50 °C, bei einem Druck von 1.013,25 mbar (Atmosphärendruck) und bei einer relativen Luftfeuchtigkeit im Bereich von 50 % bis 90 % mindestens 6 Monate, insbesondere mindestens 12 Monate, vorzugsweise mindestens 24 Monate, bevorzugt mindestens 36 Monate, stabil, insbesondere lagerstabil, ist; und/oder
wobei die Zusammensetzung (f) mindestens einen weiteren Inhaltsstoff, insbesondere Hilfsstoff und/oder Additiv, aufweist, insbesondere ausgewählt aus der Gruppe von Verarbeitungshilfsstoffen, Stabilisatoren, Emulgatoren, Antioxidantien, Feuchthaltemitteln, Verdickungsmitteln, Antiseptika, Farbstoffen, Aromastoffen, Riechstoffen, Duftstoffen, Streckmitteln, Bindemitteln, Netzmitteln, Vitaminen, Spurenelementen, Mineralstoffen, Mikronährstoffen und/oder ätherischen Ölen sowie deren Kombinationen.

9. Zusammensetzung zur Verwendung nach einem der vorangehenden Ansprüche zur Verwendung bei der prophylaktischen und/oder kurativen topischen Behandlung von Rhinitiden, insbesondere von *Rhinitis acuta*, *Rhinitis sicca*, *Rhinitis allergica*, *Rhinitis atrophicans*, *Rhinitis hyperplastica* oder *hypertrophicans*, *Rhinitis mutilans*, *Rhinitis vasomotorica*, umweltbedingter Rhinitis, *Rhinitis pseudomembranacea* oder *Rhinitis medicamentosa*, insbesondere *Rhinitis acuta*, *Rhinitis sicca* und/oder *Rhinitis allergica*.

10. Applikationsvorrichtung, insbesondere für die topische, insbesondere nasale, vorzugsweise intranasale Applikation, bevorzugt in Form eines Behältnisses mit Tropf- oder Sprüheinrichtung, enthaltend eine Zusammensetzung nach einem der vorangehenden Ansprüche.

11. Applikationsvorrichtung nach Anspruch 10, wobei die Applikationsvorrichtung eine Sprüheinrichtung zur gleichförmigen Ausbringung der Zusammensetzung in einer Menge pro Sprühstoß im Bereich von 25 µl bis 300 µl, insbesondere im Bereich von 50 µl bis 200 µl vorzugsweise im Bereich von 75 µl bis 125 µl, aufweist.

12. Applikationsvorrichtung nach Anspruch 10 oder 11, wobei die Applikationsvorrichtung einen Vorratsbehälter mit einem Volumen im Bereich von 5 ml bis 100 ml, insbesondere 10 ml bis 50 ml, aufweist.

## Claims

1. A composition for use in topical nasal, preferably intranasal, application for the treatment of rhinitises,
wherein the composition contains in combination and in effective, in particular pharmaceutically effective, amounts in each case:
(a) pantothenol, preferably dexpanthenol (D-pantothenol), or physiologically safe esters and/or pantothenic acid or physiologically safe salts thereof (component (a)), wherein the composition contains the component (a) in an amount in the range of from 0.001 to 20 wt% in relation to the composition; and
(b) cellulose and/or a physiologically safe cellulose derivative from the group of carboxymethyl cellulose, methyl cellulose, ethyl cellulose, hydroxyethyl cellulose, hydroxyethyl methyl cellulose and/or hydroxypropyl methyl cellulose (component (b)), wherein the composition contains the component (b) in an amount in the range of from 0.0001 to 5 wt% in relation to the composition;
(c) at least one preservative (component (c)), wherein the composition contains the component (c) in an amount in the range of from 0.001 to 10 wt% in relation to the composition, and wherein the component (c) is selected from the group of sorbic acid and/or pharmaceutically acceptable salts thereof (sorbates);
wherein the composition is hypertonic in relation to the cells of the nasal mucous membrane.

2. The composition for use according to claim 1,
wherein the composition contains the component (a) in the form of dexpanthenol (D-pantothenol) or physiologically safe esters thereof, preferably dexpanthenol (D-pantothenol); and/or
wherein the composition contains the component (a) in an amount in the range of from 0.005 to 15 wt%, preferably 0.01 to 10 wt%, more preferably 0.1 to 1 wt%, particularly preferably 0.2 to 0.8 wt%, in relation to the composition; and/or
wherein the cellulose derivative is hydroxypropyl methyl cellulose; and/or
wherein the composition contains the component (b) in an amount in the range of from 0.0001 to 2 wt%, preferably 0.001 to 1.5 wt%, more preferably 0.005 to 1 wt%, particularly preferably 0.01 to 0.1 wt%, even more preferably 0.02 to 0.05 wt%, in relation to the composition; and/or
wherein the composition contains the component (a) and the component (b) in a weight ratio of (a):(b) in the range of from 1:1 to 500:1, in particular 1.25:1 to 100:1, preferably 1.5:1 to 75:1, more preferably 2:1 to 50:1, particularly preferably 10:1 to 30:1.

3. The composition for use according to any one of the preceding claims,
wherein the component (c) is an alkali sorbate or alkaline earth sorbate, preferably alkali sorbate, more preferably potassium sorbate, and/or
wherein the composition contains the component (c) in an amount in the range of from 0.005 to 5 wt%, preferably 0.01 to 1 wt%, more preferably 0.1 to 0.5 wt%, in relation to the composition; and/or
wherein the composition contains the component (a) and the component (c) in a weight ratio of (a):(c) in the range of from 250:1 to 1:10, in particular 100:1 to 1:1, preferably 50:1 to 1:1, more preferably 25:1 to 1.25:1, particularly preferably 10:1 to 2:1, very particularly preferably 5:1 to 2:1, and/or
wherein the composition contains the component (b) and the component (c) in a weight ratio of (b):(c) in the range of from 1:500 to 10:1, in particular 1:250 to 5:1, preferably 1:200 to 2:1, preferably 1:50 to 1:1, particularly preferably 1:20 to 1:2, even more preferably 1:15 to 1:5.

4. The composition for use according to any one of the preceding claims,
wherein the composition (d) contains at least one chemical buffer system, in particular in the form of buffering salt(s),
in particular wherein the composition contains the chemical buffer system, in relation to the composition and calculated as a sum of all constituents of the chemical buffer system, in an amount in the range of from 0.001 to 4 wt%, in particular 0.01 to 3 wt%, preferably 0.05 to 2 wt%, more preferably 0.1 to 1.5 wt%, particularly preferably 0.2 to 1 wt%, and/or
in particular wherein the chemical buffer system is used to adjust and/or keep constant the pH value of the composition, and/or
in particular wherein the chemical buffer system exists as a dihydrogen phosphate/monohydrogen phosphate buffer system ("H₂PO₄⁻/HPO₄²⁻ buffer (system)" or "phosphate buffer (system)"), in particular as an alkali dihydrogen phosphate/alkali monohydrogen phosphate buffer system, preferably having a dihydrogen phosphate/monohydrogen phosphate molar ratio greater than 5:1, in particular in the range of from 5:1 to 200:1, preferably 6:1 to 175:1, more preferably 7:1 to 150:1, particularly preferably 8:1 to 125:1, very particularly preferably 10:1 to 100:1, even more preferably 20:1 to 90:1, and/or
in particular wherein the composition contains the component (a) and the component (d) in a weight ratio of (a):(d) in the range of from 1:100 to 100:1, in particular 1:50 to 50:1, preferably 1:20 to 20:1, more preferably 1:10 to 10:1, particularly preferably 1:5 to 2:1, very particularly preferably 1:2 to 1:1, and/or
in particular wherein the composition contains the component (b) and the component (d) in a weight ratio of (b):(d) in the range of from 1:1000 to 100:1, in particular 1:500 to 10:1, preferably 1:200 to 1:1, more preferably 1:150 to 1:15, preferably 1:100 to 1:30, and/or
in particular wherein the composition contains the component (c) and the component (d) in a weight ratio of (c):(d) in the range of from 1:500 to 100:1, in particular 1:100 to 5:1, preferably 1:20 to 3:1, more preferably 1:10 to 1:1, particularly preferably 1:6 to 1:2.

5. The composition for use according to any one of the preceding claims,
wherein the composition has a pH value in the range of from 4.5 to 8.0, in particular in the range of from 5.0 to 6.5, preferably in the range of from 5.0 to 6.2, more preferably in the range of from 5.0 to 6.0, even more preferably in the range of from 5.1 to 6.0, very particularly preferably in the range of from 5.2 to 5.9, most preferably in the range of from 5.25 to 5.85, and/or wherein the pH value of the composition is set to or kept constant at a value in the range of from 4.5 to 8.0, in particular in the range of from 5.0 to 6.5, preferably in the range of from 5.0 to 6.2, more preferably in the range of from 5.0 to 6.0, even more preferably in the range of from 5.1 to 6.0, very particularly preferably in the range of from 5.2 to 5.9, most preferably in the range of from 5.25 to 5.85, preferably by means of at least one chemical buffer system; and/or
wherein the composition (e) contains at least one physiologically safe or acceptable, osmotically active substance and/or at least one physiologically safe or acceptable osmolyte,
in particular wherein (e) the osmotically active substances and/or the osmolytes are selected from the group of ions of physiologically safe or acceptable salts (i.e. salt ions), in particular alkali ions, in particular sodium ions and/or potassium ions and/or chloride ions, preferably sodium ions and chloride ions; sugars, in particular trehalose and/or sucrose; polyols, in particular glycerol; and/or amino acids, in particular glutamic acid and/or salts thereof, proline, (hydroxy)ectoine and/or glycine betaine, and/or
in particular wherein the osmotically active substances and/or the osmolytes are sodium ions and chloride ions, in particular in the form of sodium chloride; and/or
wherein the composition (e) contains physiologically acceptable, osmotically active substances and/or physiologically acceptable osmolytes in an amount in the range of from 0.1 to 25 wt%, in particular 1 to 20 wt%, preferably 1 to 15 wt%, more preferably 1.5 to 10 wt%, particularly preferably 2 to 5 wt%, in relation to the composition.

6. The composition for use according to any one of the preceding claims,
wherein the composition contains in combination and in effective, in particular pharmaceutically effective, amounts in each case:
(a) pantothenol, preferably dexpanthenol (D-pantothenol), or physiologically safe esters and/or pantothenic acid or physiologically safe salts thereof (component (a)), in an amount in the range of from 0.001 to 20 wt%, in particular 0.005 to 15 wt%, preferably 0.01 to 10 wt%, more preferably 0.1 to 1 wt%, particularly preferably 0.2 to 0.8 wt%, in relation to the composition; and
(b) cellulose and/or at least one physiologically safe cellulose derivative (component (b)) in an amount in the range of from 0.0001 to 5 wt%, in particular 0.0001 to 2 wt%, preferably 0.001 to 1.5 wt%, more preferably 0.005 to 1 wt%, particularly preferably 0.01 to 0.1 wt%, even more preferably 0.02 to 0.05 wt%, in relation to the composition, wherein the component (b) is selected from physiologically safe cellulose derivatives and/or modified celluloses in the form of cellulose ethers and/or cellulose esters;
(c) at least one preservative (component (c)) in an amount in the range of from 0.001 to 10 wt%, in particular in the range of from 0.005 to 5 wt%, preferably in the range of from 0.01 to 1 wt%, more preferably in the range of from 0.1 to 0.5 wt%, in relation to the composition, wherein the component (c) is selected from the group of sorbic acid and/or pharmaceutically acceptable salts (sorbates) thereof;
(d) optionally at least one chemical buffer system (component (d)), in particular in the form of buffer salt(s), in particular in an amount in the range of from 0.001 to 4 wt%, in particular 0.01 to 3 wt%, preferably 0.05 to 2 wt%, more preferably 0.1 to 1.5 wt%, particularly preferably 0.2 to 1 wt%, in relation to the composition and calculated as the sum of all constituents of the chemical buffer system; and
(e) optionally at least one physiologically safe or acceptable, osmotically active substance and/or physiologically safe or acceptable osmolytes (component (e)) in an amount in the range of from 0.1 to 25 wt%, in particular 1 to 20 wt%, preferably 1 to 15 wt%, more preferably 1.5 to 10 wt%, particularly preferably 2 to 5 wt%, in relation to the composition.

7. The composition for use according to any one of the preceding claims,
wherein the composition exists as an aqueous composition and/or wherein the composition is aqueous-based and/or has an aqueous formulation, in particular in the form of an aqueous solution or aqueous solubilisation; and/or
wherein the composition contains water, in particular purified water, in an amount in the range of from 60 to 99 wt%, in particular in the range of from 70 to 98 wt%, more preferably in the range of from 80 to 97 wt%, particularly preferably in the range of from 90 to 96 wt%, in relation to the composition, and/or wherein the composition contains water as a pharmaceutically acceptable carrier (excipient) and/or wherein the composition is aqueous-based; and/or
wherein the composition has an osmolality in the range of from 300 to 600 mOsm/kg, in particular in the range of from 310 to 550 mOsm/kg, preferably in the range of from 300 to 525 mOsm/kg, more preferably in the range of from 325 to 510 mOsm/kg, particularly preferably in the range of from 350 to 500 mOsm/kg, and/or
wherein the composition at a temperature of 20°C and at a pressure of 1013.25 mbar (atmospheric pressure) has a relative density, in relation to pure water, in the range of from 1.001 to 1.5, in particular in the range of from 1.001 to 1.2, preferably in the range of from 1.005 to 1.15, more preferably in the range of from 1.005 to 1.1.

8. The composition for use according to any one of the preceding claims,
wherein the composition at a temperature of 20°C has a dynamic viscosity of at least 1.1 mPas, in particular in the range of from 1.1 to 10⁵ mPas, preferably in the range of from 1.2 to 10⁴ mPas, particularly preferably in the range of from 1.3 to 10³ mPas, even more preferably in the range of from 1.5 to 100 mPas (in particular determined in accordance with the method according to European Pharmacopoeia (Ph.Eur.) 7th edition, 2011, section 2.2.9 "Capillary viscometer"); and/or
wherein the composition, at temperatures in the range of from 20°C to 50°C, at a pressure of 1013.25 mbar (atmospheric pressure) and at a relative air humidity in the range of from 50% to 90%, is stable, in particular is stable in storage, for at least 6 months, in particular at least 12 months, preferably at least 24 months, more preferably at least 36 months; and/or
wherein the composition (f) comprises at least one further ingredient, in particular an adjuvant and/or additive, in particular selected from the group of processing aids, stabilisers, emulsifiers, antioxidants, humectants, thickeners, antiseptics, dyes, flavourings, fragrances, perfumes, extenders, binders, wetting agents, vitamins, trace elements, minerals, micronutrients and/or essential oils and combinations thereof.

9. The composition for use according to any one of the preceding claims for use in the prophylaxis and/or curative topical treatment of rhinitises, in particular of *rhinitis acuta*, *rhinitis sicca*, *rhinitis allergica*, *rhinitis atrophicans*, *rhinitis hyperplastica* or *hypertrophicans*, *rhinitis mutilans*, *rhinitis vasomotorica*, environment-induced rhinitis, *rhinitis pseudomembranacea* or *rhinitis medicamentosa*, in particular *rhinitis acuta*, *rhinitis sicca* and/or *rhinitis allergica.*

10. An application device, in particular for topical, in particular nasal, preferably intranasal, application, more preferably in the form of a container having a dropper or spraying means, containing a composition according to any of the preceding claims.

11. The application device according to claim 10, wherein the application device has a spraying means for uniformly dispensing the composition in an amount per spray in the range of from 25 µl to 300 µl, in particular in the range of from 50 µl to 200 µl, preferably in the range of from 75 µl to 125 µl.

12. The application device according to claim 10 or 11, wherein the application device has a storage container having a volume in the range of from 5 ml to 100 ml, in particular 10 ml to 50 ml.

## Revendications

1. Composition destinée à être utilisée en administration topique nasale, de préférence intranasale pour le traitement de rhinites,
la composition en association et pour chacun en des quantités efficaces, notamment pharmaceutiquement efficaces, contenant :
(a) du pantothénol, de préférence du dexpanthénol (D-pantothénol) ou ses esters physiologiquement acceptables et/ou de l'acide pantothénique ou ses sels physiologiquement acceptables (constituant (a)), la composition contenant le constituant (a) en une quantité dans la plage de 0,001 à 20 % en poids par rapport à la composition ; et
(b) de la cellulose et/ou un dérivé de cellulose physiologiquement acceptable choisi parmi le groupe constitué par la carboxyméthylcellulose, la méthylcellulose, l'éthylcellulose, l'hydroxyéthylcellulose, l'hydroxyéthylméthylcellulose et/ou l'hydroxypropylméthylcellulose (constituant (b)), la composition contenant le constituant (b) en une quantité dans la plage de 0,0001 à 5 % en poids par rapport à la composition ;
(c) au moins un agent de conservation (constituant (c)), la composition contenant le constituant (c) en une quantité dans la plage de 0,001 à 10 % en poids, par rapport à la composition, et le constituant (c) étant choisi dans le groupe constitué par l'acide sorbique et/ou ses sels pharmaceutiquement acceptables (sorbates) ;
la composition étant formée de manière hypertonique contre les cellules de la muqueuse nasale.

2. Composition destinée à être utilisée selon la revendication 1,
la composition contenant le constituant (a) sous la forme de dexpanthénol (D-pantothénol) ou ses esters physiologiquement acceptables, de préférence de dexpanthénol (D-pantothénol) ; et/ou
la composition contenant le constituant (a) en une quantité dans la plage de 0,005 à 15 % en poids, de préférence de 0,01 à 10 % en poids, préférablement de 0,1 à 1 % en poids, de manière particulièrement préférée de 0,2 à 0,8 % en poids, par rapport à la composition ; et/ou
le dérivé de cellulose étant l'hydroxypropylméthylcellulose ; et/ou
la composition contenant le constituant (b) en une quantité dans la plage de 0,0001 à 2 % en poids, de préférence de 0,001 à 1,5 % en poids, préférablement de 0,005 à 1 % en poids, de manière particulièrement préférée de 0,01 à 0,1 % en poids, plus préférablement de 0,02 à 0,05 % en poids par rapport à la composition ; et/ou
la composition contenant le constituant (a) et le constituant (b) dans un rapport pondéral (a):(b) dans la plage de 1:1 à 500:1, en particulier de 1,25:1 à 100:1, de préférence de 1,5:1 à 75:1, préférentiellement de 2:1 à 50:1, préférablement de 10:1 à 30:1.

3. Composition destinée à être utilisée selon l'une des revendications précédentes,
le constituant (c) étant un sorbate de métal alcalin ou alcalino-terreux, de préférence un sorbate de métal alcalin, préférablement le sorbate de potassium et/ou
la composition contenant le constituant (c) en une quantité dans la plage allant de 0,005 à 5 % en poids, de préférence dans la plage de 0,01 à 1 % en poids, préférablement dans la plage de 0,1 à 0,5 % en poids, par rapport à la composition ; et/ou
la composition contenant le constituant (a) et le constituant (c) dans un rapport pondéral (a):(c) dans la plage de 250:1 à 1:10, en particulier de 100:1 à 1:1, de préférence de 50:1 à 1:1, préférentiellement de 25:1 à 1,25:1, préférablement de 10:1 à 2:1, préférablement de 5:1 à 2:1 et/ou
la composition contenant le constituant (b) et le constituant (c) dans un rapport pondéral (b):(c) dans la plage de 1:500 à 10:1, en particulier de 1:250 à 5:1, de préférence de 1:200 à 2:1, préférentiellement de 1:50 à 1:1, préférablement de 1:20 à 1:2, encore plus préférablement de 1:15 à 1:5.

4. Composition destinée à être utilisée selon l'une des revendications précédentes,
la composition (d) contenant au moins un système tampon chimique, notamment sous forme d'un ou plusieurs sels tampons,
en particulier, la composition contenant le système tampon chimique, par rapport à la composition et calculé sous forme de somme de tous les composants du système tampon chimique, en une quantité dans la plage de 0,001 à 4 % en poids, en particulier de 0,01 à 3 % en poids, de préférence de 0,05 à 2 % en poids, préférablement de 0,1 à 1,5 % en poids, de manière particulièrement préférée de 0,2 à 1 % en poids et/ou
en particulier, le système tampon chimique servant à ajuster et/ou à maintenir constante le pH de la composition et/ou
en particulier, le système tampon chimique étant présent sous la forme d'un système tampon dihydrogénophosphate/monohydrogénophosphate (« système tampon ou « (système) tampon phosphate» H₂PO₄⁻/HPO₄²⁻ »), en particulier sous la forme d'un système tampon dihydrogénophosphate de métal alcalin/monohydrogénophosphate de métal alcalin, de préférence avec un rapport molaire dihydrogénophosphate/monohydrogénophosphate supérieur à 5:1, en particulier dans la plage de 5:1 à 200:1, de préférence de 6:1 à 175:1, préférentiellement de 7:1 à 150:1, préférablement de 8:1 à 125:1, préférablement de 10:1 à 100:1, encore plus préférablement de 20:1 à 90:1 et/ou
en particulier, la composition contenant le constituant (a) et le constituant (d) dans un rapport pondéral (a):(d) dans la plage de 1:100 à 100:1, en particulier de 1:50 à 50:1, de préférence de 1:20 à 20:1, préférentiellement de 1:10 à 10:1, préférablement de 1:5 à 2:1, préférablement de 1:2 à 1:1 et/ou
en particulier, la composition contenant le constituant (b) et le constituant (d) dans un rapport pondéral (b):(d) dans la plage de 1:1000 à 100:1, en particulier de 1:500 à 10:1, de préférence de 1:200 à 1:1, préférentiellement de 1:150 à 1:15, de préférence de 1:100 à 1:30 et/ou
en particulier, la composition contenant le constituant (c) et le constituant (d) dans un rapport pondéral (c):(d) dans la plage de 1:500 à 100:1, en particulier de 1:100 à 5:1, de préférence de 1:20 à 3:1, préférentiellement de 1:10 à 1:1, préférablement de 1:6 à 1:2.

5. Composition destinée à être utilisée selon l'une des revendications précédentes,
la composition présentant un pH dans la plage de 4,5 à 8,0, en particulier dans la plage de 5,0 à 6,5, de préférence dans la plage de 5,0 à 6,2, préférablement dans la plage de 5,0 à 6,0, plus préférablement dans la plage de 5,1 à 6,0, encore plus préférablement dans la plage de 5,2 à 5,9, le plus préférablement dans la plage de 5,25 à 5,85 et/ou le pH de la composition étant ajustée et/ou maintenue constante à une valeur dans la plage de 4,5 à 8,0, en particulier dans la plage de 5,0 à 6,5, de préférence dans la plage de 5,0 à 6,2, préférablement dans la plage de 5,0 à 6,0, plus préférablement dans la plage de 5,1 à 6,0, encore plus préférablement dans la plage de 5,2 à 5,9, le plus préférablement dans la plage de 5,25 à 5,85, de préférence au moyen d'au moins un système tampon chimique ; et/ou
la composition contenant (e) au moins une substance physiologiquement acceptable ou inoffensive, osmotiquement active et/ou au moins un osmolyte physiologiquement acceptable ou inoffensive,
en particulier (e) la substance osmotiquement active et/ou l'osmolyte étant choisis dans le groupe d'ions de sels physiologiquement acceptables ou inoffensifs (c'est-à-dire des ions de sels), en particulier des ions de métaux alcalins, en particulier des ions sodium et/ou potassium et/ou des ions chlorure, de préférence des ions sodium et des ions chlorure ; des sucres, en particulier le tréhalose et/ou le saccharose ; des polyols, en particulier le glycérol ; et/ou des acides aminés, en particulier l'acide glutamique et/ou ses sels, la proline, l'(hydroxy-)ectoïne et/ou la glycine-bétaïne et/ou
en particulier, la substance osmotiquement active et/ou l'osmolyte étant des ions sodium et des ions chlorure, en particulier sous la forme de chlorure de sodium ; et/ou la composition contenant (e) une substance osmotiquement active physiologiquement acceptable et/ou un osmolyte physiologiquement acceptable en une quantité dans la plage de 0,1 à 25 % en poids, en particulier de 1 à 20 % en poids, de préférence de 1 à 15 % en poids, préférablement de 1,5 à 10 % en poids, de manière particulièrement préférée de 2 à 5 % en poids, par rapport à la composition.

6. Composition destinée à être utilisée selon l'une des revendications précédentes, la composition en association et pour chacun en des quantités efficaces, notamment pharmaceutiquement efficaces, contenant :
(a) du pantothénol, de préférence du dexpanthénol (D-pantothénol), ou ses esters physiologiquement acceptables et/ou de l'acide pantothénique ou ses sels physiologiquement acceptables (constituant (a)) en une quantité dans la plage de 0,001 à 20 % en poids, en particulier de 0,005 à 15 % en poids, de préférence de 0,01 à 10 % en poids, préférablement de 0,1 à 1 % en poids, de manière particulièrement préférée de 0,2 à 0,8 % en poids, par rapport à la composition ; et
(b) de la cellulose et/ou au moins un dérivé de cellulose physiologiquement acceptable (constituant (b)) en une quantité dans la plage de 0,0001 à 5 % en poids, en particulier de 0,0001 à 2 % en poids, de préférence de 0,001 à 1,5 % en poids, préférablement de 0,005 à 1 % en poids, de manière particulièrement préférée de 0,01 à 0,1 % en poids, plus préférablement de 0,02 à 0,05 % en poids, par rapport à la composition, le constituant (b) étant choisi parmi des dérivés de cellulose physiologiquement acceptables et/ou des celluloses modifiées sous la forme d'éthers de cellulose et/ou d'esters de cellulose ;
(c) au moins un agent de conservation (constituant (c)) en une quantité dans la plage de 0,001 à 10 % en poids, en particulier dans la plage de 0,005 à 5 % en poids, de préférence dans la plage de 0,01 à 1 % en poids, préférablement dans la plage de 0,1 à 0,5 % en poids, par rapport à la composition, le constituant (c) étant choisi dans le groupe constitué par l'acide sorbique et/ou ses sels pharmaceutiquement acceptables (sorbates) ;
(d) le cas échéant au moins un système tampon chimique (constituant (d)), en particulier sous la forme d'un ou de plusieurs sels tampons, en particulier en une quantité dans la plage de 0,001 à 4 % en poids, en particulier de 0,01 à 3 % en poids, de préférence de 0,05 à 2 % en poids, préférablement de 0,1 à 1,5 % en poids, de manière particulièrement préférée de 0,2 à 1 % en poids, par rapport à la composition et calculé sous forme de somme de tous les composants du système tampon chimique ; et
(e) le cas échéant au moins une substance osmotiquement active physiologiquement acceptable ou inoffensive et/ou des osmolytes physiologiquement acceptables ou inoffensifs (constituant (e)) en une quantité dans la plage de 0,1 à 25 % en poids, en particulier de 1 à 20 % en poids, de préférence de 1 à 15 % en poids, préférablement de 1,5 à 10 % en poids, de manière particulièrement préférée 2 à 5 % en poids, par rapport à la composition.

7. Composition destinée à être utilisée selon l'une des revendications précédentes,
la composition étant présente sous la forme d'une composition aqueuse et/ou la composition ayant une base aqueuse et/ou se présentant sous la forme d'une formulation aqueuse, en particulier sous la forme d'une solution aqueuse ou d'une solubilisation aqueuse ; et/ou
la composition contenant de l'eau, en particulier de l'eau purifiée, dans une quantité située dans la plage de 60 à 99 % en poids, en particulier dans la plage de 70 à 98 % en poids, de manière davantage préférée dans la plage de 80 à 97 % en poids, de manière particulièrement préférée dans la plage de 90 à 96 % en poids, par rapport à la composition, et/ou la composition contenant de l'eau en tant que véhicule (excipient) pharmaceutiquement acceptable et/ou la composition étant conçue sur une base aqueuse ; et/ou
la composition ayant une osmolalité dans la plage de 300 à 600 mOsm/kg, en particulier dans la plage de 310 à 550 mOsm/kg, de préférence dans la plage de 300 à 525 mOsm/kg, de préférence dans la plage de 325 à 510 mOsm/kg, de préférence encore dans la plage de 350 à 500 mOsm/kg ; et/ou
la composition présentant, à une température de 20 °C et à une pression de 1013,25 mbar (pression atmosphérique) une densité relative, par rapport à l'eau pure, dans la plage de 1,001 à 1,5, en particulier dans la plage de 1,001 à 1,2, de préférence dans la plage de 1,005 à 1,15, préférablement dans la plage de 1,005 à 1,1.

8. Composition destinée à être utilisée selon l'une des revendications précédentes,
la composition présentant, à une température de 20 °C, une viscosité dynamique d'au moins 1,1 mPa.s, en particulier dans la plage de 1,1 à 10⁵mPa.s, de préférence dans la plage de 1,2 a 10⁴ mPa.s, de manière particulièrement préférée dans la plage de 1,3 à 10³ mPa.s, plus préférablement dans la plage de 1,5 à 100 mPa.s (en particulier déterminée selon la méthode Ph.Eur. 7ème édition. texte de base 2011, monographie 2.2.9 « viscosimètre capillaire ») ; et/ou
la composition étant stable, en particulier stable au stockage, à des températures dans la plage de 20 °C à 50 °C, à une pression de 1013,25 mbar (pression atmosphérique) et à une humidité relative de l'air dans la plage de 50 % à 90 %, pendant au moins 6 mois, en particulier au moins 12 mois, de préférence au moins 24 mois, préférablement au moins 36 mois ; et/ou
la composition présentant (f) au moins un autre ingrédient, en particulier un adjuvant et/ou un additif, notamment choisi dans le groupe des auxiliaires de mise en oeuvre, des stabilisants, des émulsifiants, des antioxydants, des humectants, des épaississants, des antiseptiques, des colorants, des arômes, des substances odoriférantes, des parfums, des diluants, des liants, des agents mouillants, des vitamines, des oligo-éléments, des minéraux, des micronutriments et/ou des huiles essentielles et de leurs combinaisons.

9. Composition destinée à être utilisée selon une des revendications précédentes, destinée à être utilisée pour le traitement prophylactique et/ou curatif de rhinites, en particulier de *Rhinitis acuta*, *Rhinitis sicca*, *Rhinitis allergica*, *Rhinitis atrophicans*, *Rhinitis hyperplastica* ou *hypertrophicans*, *Rhinitis mutilans*, *Rhinitis vasomotorica*, Rhinitis environnementale, *Rhinitis pseudomembranacea* ou *Rhinitis medicamentosa*, en particulier *Rhinitis acuta*, *Rhinitis sicca* et/ou *Rhinitis allergica.*

10. Dispositif d'administration, en particulier pour l'administration topique, notamment nasale, de préférence intranasale, préférablement sous la forme d'un contenant doté d'un compte-goutte ou d'un pulvérisateur, contenant une composition selon l'une des revendications précédentes.

11. Dispositif d'administration selon la revendication 10, le dispositif d'administration présentant un pulvérisateur pour la distribution homogène de la composition en une quantité par décharge dans la plage de 25 µl à 300 µl, en particulier dans la plage de 50 µl à 200 µl, de préférence dans la plage de 75 µl à 125 µl.

12. Dispositif d'administration selon la revendication 10 ou 11, le dispositif d'administration présentant un réservoir ayant un volume dans la plage de 5 ml à 100 ml, en particulier de 10 ml à 50 ml.
